# EUROPEAN PATENT SPECIFICATION

(11) **EP 0 621 265 B1**
(45) Date of publication and mention of the grant of the patent: **22.01.1997**
(21) Application number: 94106334.9
(22) Date of filing: 22.04.1994
(51) Int. Cl.: C07C 305/10, C11D 1/12

(54) **N-alkylcarbamylalkanol sulfate or salt thereof, process for producing the same and detergent composition containing the same**
N-Alkylcarbamylalkanolsulfat oder deren Salz, deren Verfahren zur Herstellung sowie dieses enthaltende Detergenszusammensetzung
Sulfate de N-alkylcarbamylalkanol ou son sel, son procédé de préparation et composition détergent le contenant

(30) Priority: 23.04.1993 JP 97848/93
(43) Date of publication of application: 26.10.1994
(73) Proprietor: KAO CORPORATION, Chuo-ku, Tokyo (JP)
(72) Inventor: Mizushima, Hiromoto, Wakayama-shi, Wakayama (JP); Takahashi, Masakatsu, Wakayama-shi, Wakayama (JP); Yamamuro, Akira, Wakayama-shi, Wakayama (JP); Matsuo, Takashi, Ichikawa-shi Chiba (JP)
(74) Representative: Hansen, Bernd, Dr. Dipl.-Chem.

(56) References cited:
- GB-A- 499 022
- US-A- 2 120 512
- US-A- 2 255 082

## Description

The present invention relates to an N-alkyl-carbamylalkanol sulfate and a salt thereof, which has excellent foaming power and detergency while being low in irritancy to the skin, so as to be widely useful as detergents for hair, skin, tableware, etc. The present invention also relates to a process for producing an N-alkyl-carbamylalkanol sulfate and a salt thereof and a detergent composition containing the same.

### Description of the Related Art

In recent years, it is demanded that the surfactant used as a detergent have excellent surface activation capability and other properties, such as biodegradability and safety to the skin and eyes.

Surfactants satisfying the above demand include an acylated amino acid surfactant, an imidazoline surfactant and a saccharide surfactant such as an alkyl glycoside, which are widely used. However, these surfactants are generally poor in detergency and foaming power, which are especially important in a detergent. Therefore, their sole use as an ingredient in a shampoo or the like is uncommon, and they are generally used in combination with conventional anionic surfactants, such as an alkyl sulfate and an alkyl ether sulfate.

On the other hand, a sulfate of a monoethanolamide of a fatty acid and an isethionic ester are surfactants that are excellent in both safety and foaming power. However, these surfactants are unstable in solution.

Therefore, the development of a detergent having not only excellent foaming power and detergency, but also high safety and stability is strongly demanded in the art.

GB-A-499 022 refers to carboxylic amides being useful as assistants in the textile industry.

US-A-2 255 082 discloses carboxylic amides as treating materials for textiles, leather and paper.

In US-A-2 120 512 specific sulphonated oxy fatty acid-amides are described as being useful as soap substitutes.

### Disclosure of the Invention

### Summary of the Invention

In view of the above situation, the present inventors have made extensive studies to develop a surfactant having not only excellent foaming power and detergency, but also high safety so as to be useful as a detergent for hair, body, tableware, etc. As a result, they have found that an N-alkylcarbamylalkanol sulfate having an amide group derived from a primary or secondary amine of a long chain and a sulfate group (-OSO₃⁻), and a salt thereof are surfactants satisfying the above demand. Based on this finding, the present invention has been completed.

Thus, the present invention provides a N-alkylcarbamylalkanol sulfate or a salt thereof represented by the general formulae (1-1) or (1-2), a process for producing the same, and a detergent composition comprising the same, which has not only excellent foaming power and detergency but also high safety. 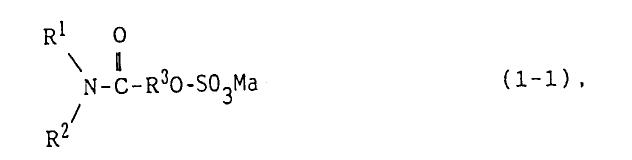 and 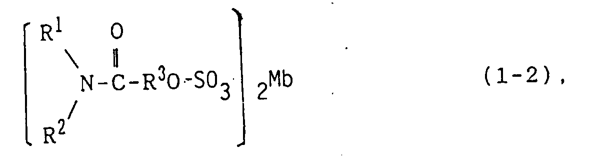 wherein R¹ represents a linear or branched alkyl or alkenyl group having 6 to 22 carbon atoms; R² represents a methyl group or a hydrogen atom: R³ represents a linear or branched alkylene group having 1 to 5 carbon atoms; Ma represents a hydrogen atom, an alkali metal atom, an ammonium group, an alkanolammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group or a group consisting of a basic amino acid and a hydrogen atom; and Mb represents an alkaline earth metal atom; under the proviso that a compound of the formula (1-1) with R¹ being represented by a C₁₉ alkyl group of the formula 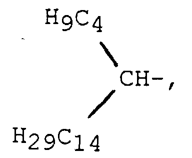 R² being represented by a hydrogen atom, R³ being represented by a methylene group and Ma being represented by a sodium atom is excluded.

Preferable N-alkylcarbamylalkanol sulfates and salts thereof according to the present invention include those belonging to the following groups A to D.
A. Salts of N-alkylcarbamylalkanol sulfates represented by the above general formula (1-1), wherein R¹ represents a linear alkyl group having 8 to 18 carbon atoms; R² represents a hydrogen atom or a methyl group; R³ represents a linear alkylene group having 1 to 5 carbon atoms; and Ma represents an ammonium group, sodium or a triethanolammonium group.
B. Salts of N-alkylcarbamylalkanol sulfates represented by the above general formula (1-1), wherein R¹ represents a linear alkyl group having 8 to 18 carbon atoms; R² represents a hydrogen atom; R³ represents a linear or branched alkylene group having 1 to 5 carbon atoms; and Ma represents an ammonium group, sodium or a triethanolammonium group.
C. Salts of N-alkylcarbamylalkanol sulfates represented by the above general formula (1-1), wherein R¹ represents a linear alkyl group having 8 to 18 carbon atoms; R² represents a hydrogen atom; R³ represents a methylene group, a pentamethylene group or a 2-methylbutylene group; and Ma represents an ammonium group, sodium or a triethanolammonium group.
D. Salts of N-alkylcarbamylalkanol sulfates represented by the above general formula (1-1), wherein R¹ represents a linear alkyl group having 8 to 18 carbon atoms; R² represents a hydrogen atom; R³ represents a methylene group; and Ma represents an ammonium group, sodium or a triethanolammonium group.

The invention furthermore includes a detergent composition comprising the N-alkylcarbamylalkanol sulfate or a salt thereof represented by the above formula (1-1) or (1-2).

According to a further aspect of the invention, there are also included detergent compositions comprising
(1) 1 to 70% by weight, based on the total weight of the composition, of N-alkylcarbamylalkanol sulfate or a salt thereof represented by the general formula (2-1) or (2-2) 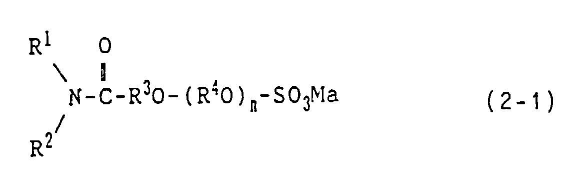 and 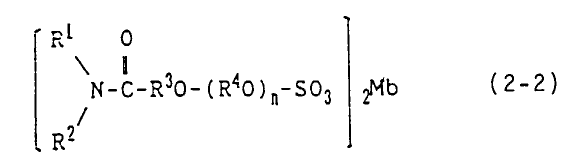 wherein R¹ represents a linear or branched alkyl or alkenyl group having 6 to 22 carbon atoms; R² represents a C₁-C₂₂ alkyl group, a C₂-C₂₂ alkenyl group or a hydrogen atom; R³ represents a linear or branched alkylene group having 1 to 5 carbon atoms; R⁴0 represents an oxyalkylene group having 2 or 3 carbon atoms; n represents an average addition mole number of and oxyalkylene group and is a number between 0 and 20, and wherein each R₄O group may be the same or different from one another; Ma represents a hydrogen atom, an alkali metal atom, an ammonium group, an alkanol-ammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group, or a group consisting of a basic amino acid and a hydrogen atom; and Mb represents an alkaline earth metal atom; and
(2) 0.01 to 3% by weight, based on the total weight of the composition, of a silicone derivative, and/or 0.05 to 10% by weight, based on the total weight of composition, of a cationic surfactant, and/or 0.01 to 3% by weight, based in the total weight of the composition, of a water-soluble cationic polymer.

Further scope and applicability of the present invention will become apparent from the detailed description given hereinafter.

### Detailed Description of the Invention

First, the N-alkylcarbamylalkanol sulfates and salts thereof according to the present invention are described.

Although in the general formulae (1-1) (1-2) (2-1) and (2-2), R¹ represents a linear or branched alkyl group or alkenyl group having 6 to 22 carbon atoms, R¹ preferably represents a linear or branched alkyl group having 6 to 22 carbon atoms, and more preferably a linear alkyl group having 8 to 18 carbon atoms, from the viewpoint of foaming power of surfactants represented by these formulae. Examples of alkyl groups of R¹ include hexyl group, octyl group, decyl group, dodecyl group, tetradecyl group, hexadecyl group, octadecyl group, isostearyl group, eicosyl group and docosyl group, while examples of alkenyl groups of R¹ include octadecenyl group.

Although R² represents an alkyl or alkenyl group having 1 to 22 carbon atoms or a hydrogen atom in the formula (2-1) or (2-2), R² preferably represents a hydrogen atom or a methyl group, and more preferably a hydrogen atom, from the viewpoints of foaming power, safety and chemical stability of surfactants represented by the general formulae (2-1) or (2-2). Examples of alkyl groups of R² include a methyl group, an ethyl group, a propyl group, a butyl group and alkyl groups mentioned as examples of R¹, while examples of alkenyl groups of R¹ include octadecenyl group.

Although R³ represents a linear or branched alkylene group having 1 to 5 carbon atoms, R³ preferably represents a linear or branched alkylene group having 2 to 5 carbon atoms, and more preferably a linear or branched alkylene group having 3 to 5 carbon atoms. Specific examples of the alkylene groups include a methylene group, a pentamethylene group (pentylene group) and a 2-methylbutylene group. A methylene group is preferred from the viewpoints of foaming power and chemical stability of surfactants represented by the general formulae (1-1), (1-2), (2-1) or (2-2) - R⁴O represents an oxyalkylene group having 2 or 3 carbon atoms. That is, R⁴O represents -CH₂CH₂O-, -CH₂CH₂CH₂O- or -CH₂CH(CH₃)O-, and preferably -CH₂CH₂O- (oxyethylene group) or -CH₂CH(CH₃)O- (oxypropylene group). The oxyalkylene group may be only one oxyalkylene group or a mixture of at least two oxyalkylene groups.

n represents an average value of the number of oxyalkylene groups which are contained in a surfactant represented by the general formulae (2-1) or (2-2) (an average addition mole number of alkyleneoxide group). n is a number between 0 and 20, preferably between 0 and 10 and more preferably 0.

Ma in the general formula (1-1) or (2-1) represents a hydrogen atom, an alkali metal atom, an ammonium group, an alkanolammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group or a group consisting of a basic amino acid and a hydrogen atom. From the viewpoint of foaming power of the surfactant, Ma preferably represents an ammonium group, sodium, potassium or an alkanolammonium group having 2 to 9 carbon atoms in total and still more preferably an ammonium group, sodium or a triethanolammonium group.

Mb in the general formula (1-2) or (2-2) represents an alkaline earth metal atom.

Suitable examples of salts of N-alkylcarbamylalkanol sulfate represented by the above general formulae (1-1), (1-2), (2-1) or (2-2) according to the present invention include:
CH₃(CH₂)₁₁NHCOCH₂OSO₃NH₄, CH₃(CH₂)₁₁NHCO(CH₂)₅OSO₃NH₄, CH₃(CH₂)₉NHCOCH₂OSO₃NH₄, CH₃(CH₂)₁₁N(CH₃)COCH₂OSO₃Na, CH₃(CH₂)₁₁NHCOCH₂OSO₃Na, CH₃(CH₂)₁₁NHCO(CH₂)₂OSO₃Na, CH₃(CH₂)₁₁NHCO(CH₂)₃OSO₃Na, CH₃(CH₂)₁₁NHCO(CH₂)₄OSO₃Na, CH₃(CH₂)₁₁NHCO(CH₂)₅OSO₃Na, CH₃(CH₂)₁₁NHCOCH₂CH(CH₃)CH₂CH₂OSO₃Na, CH₃(CH₂)₁₁NHCOCH₂CH(CH₃)CH₂CH₂OSO₃K, CH₃(CH₂)₁₃NHCO(CH₂)₃OSO₃Na, CH₃(CH₂)₁₃NHCO(CH₂)₃OSO₃NH₄, CH₃(CH₂)₁₃NHCO(CH₂)₄OSO₃NH₄, CH₃(CH₂)₉NHCO(CH₂)₃OSO₃Na, CH₃(CH₂)₉NHCO(CH₂)₃OSO₃HN(CH₂CH₂OH)₃, CH₃(CH₂)₉NHCO(CH₂)₃OSO₃H₂N(CH₂CH₂OH)₂, CH₃(CH₂)₉NHCO(CH₂)₃OSO₃H₃NCH₂CH₂OH, CH₃(CH₂)₉NHCO(CH₂)₅OSO₃Na, CH₃(CH₂)₇NHCO(CH₂)₄OSO₃Na, CH₃(CH₂)₁₁N(CH₃)CO(CH₂)₃OSO₃Na, CH₃(CH₂)₁₃N(CH₃)CO(CH₂)₄OSO₃Na, CH₃(CH₂)₁₁NHCO(CH₂)₃OCH₂CH₂OSO₃Na, CH₃(CH₂)₁₁NHCO(CH₂)₄OCH₂CH(CH₃)OSO₃Na, and CH₃(CH₂)₉NHCO(CH₂)₄O(CH₂CH₂O)₅SO₃Na.

Next, the process for producing the N-alkylcarbamylalkanol sulfate or the salt thereof according to the present invention is described.

The N-alkylcarbamylalkanol sulfate or the salt thereof represented by the general formulae (1-1) or (1-2) according to the present invention can be easily produced by conducting steps selected from A to C in an order selected from the group consisting of (1) A and B, and (2) C and B:

### step A

Reacting an aliphatic amine represented by the general formula (2): 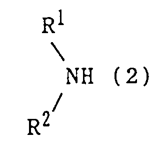 (wherein R¹ and R² are defined above), with a cyclic lactone represented by the general formula (3): 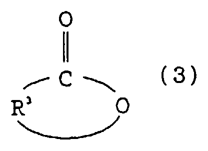 (wherein R³ is defined above), to thereby obtain an N-alkylcarbamylalkanol represented by the general formula (4): 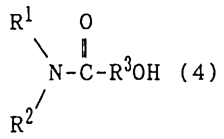 (wherein R¹, R² and R³ are defined above);

### step B

Reacting the N-alkylcarbamylalkanol represented by the above general formulae (4) with a sulfating agent to thereby obtain an N-alkylcarbamylalkanol sulfate represented by the above general formula (1-1) (with the proviso that Ma is a hydrogen atom), and, if necessary, neutralizing the N-alkylcarbamylalkanol sulfate thus obtained with a basic compound to thereby obtain a salt of N-alkylcarbamylalkanol sulfate represented by the above general formulae (1-1) (with the proviso that Ma is not a hydrogen atom) or (1-2); and

### step C

Reacting the aliphatic amine represented by the above general formula (2) with an ω-hydroxycarboxylic acid or an ester thereof represented by the general formula (6): HO-R³CO₂R⁵ (6) (wherein R³ is defined above, and R⁵ represents a hydrogen atom or a linear or branched alkyl group having 1 to 4 carbon atoms) to thereby obtain an N-alkylcarbamylalkanol represented by the above general formula (4).

The process for producing the N-alkylcarbamylalkanol sulfate, i.e., the N-alkyl-amidoalkanol sulfate, or the salt thereof of the formula (2-1) or (2-2) comprises conducting the following steps A', B' and C'; A' and C'; D', B' and C'; or D' and C' in this order:

### step A'

Reacting an aliphatic amine represented by the general formula (2): 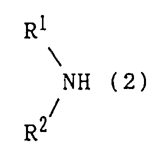 (wherein R¹ and R² are defined above), with a cyclic lactone represented by the general formula (3): 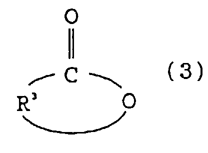 (wherein R³ is defined above), to thereby obtain an N-alkylamidoalkanol represented by the general formula (4): 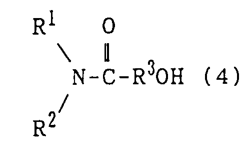 (wherein R¹, R² and R³ are defined above);

### step B'

Reacting the N-alkylamidoalkanol represented by the above general formula (4) with an alkylene oxide in the presence of an alkali or acid catalyst to thereby obtain an N-alkylamidoalkanol represented by the general formula (5): 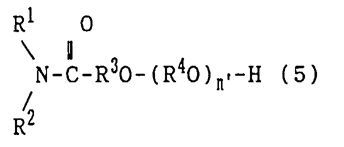 (wherein R¹, R², R³ and R⁴ are defined above, and n' represents a number of 1 to 20);

### step C'

Reacting the N-alkylamidoalkanol represented by the above general formulae (4) or (5) with a sulfating agent, and then neutralizing with a basic compound and

### step D'

Reacting the aliphatic amine represented by the above general formula (2) with an ω-hydroxycarboxylic acid or an ester thereof represented by the general formula (6): HO-R³CO₂R⁵ (6) (wherein R³ is as defined above, and R⁵ represents a hydrogen atom or a linear or branched alkyl group having 1 to 4 carbon atoms) to thereby obtain an N-alkylamidoalkanol represented by the above general formula (4).

Hereinbelow, each of the above steps will be described in greater detail.

### step A or A'

This is a step in which an aliphatic amine represented by the above general formula (2) (hereinafter referred to simply as "aliphatic amine (2)") is reacted with a cyclic lactone represented by the above general formula (3) (hereinafter referred to simply as "cyclic lactone (3)") to thereby obtain an N-alkylcarbamylalkanol represented by the above general formula (4) (hereinafter referred to simply as "N-alkylcarbamylalkanol (4)").

The aliphatic amine (2) and cyclic lactone (3) as the starting compounds may be either those produced by known processes, or those commercially available. If desired, those purified by recrystallization, distillation or the like may be used.

Examples of aliphatic amines (2) include octylamine, decylamine, dodecylamine, tetradecylamine, hexadecylamine, octadecylamine, octylmethylamine, decylmethylamine, dodecylmethylamine, tetradecylmethylamine, hexadecylmethylamine and octadecylmethylamine. Among them, octylamine, decylamine, dodecylamine, tetradecylamine, hexadecylamine and octadecylamine are preferred, and octylamine, decylamine, dodecylamine and tetradecylamine are particularly preferred.

Examples of cyclic lactones (3) include β-propiolactone, γ-butyrolactone, δ-valerolactone, 2-methyl-δ-valerolactone, ε-caprolactone and 2-methyl-γ-butyrolactone. Among them, γ-butyrolactone, δ-valerolactone, 2-methyl-δ-valerolactone and ε-caprolactone are preferred, and 2-methyl-δ-valerolactone and ε-caprolactone are particularly preferred.

In this step, the N-alkylcarbamylalkanol (4) can be obtained by reacting an aliphatic amine (2) with a cyclic lactone (3) in an amount of 1 to 1.5 times by mol that of the aliphatic amine (2) in the absence of any solvent, or, if necessary, in a solvent such as toluene, xylene, dioxane and DMF, at an appropriate temperature between 0 and 180°C, preferably between 70 and 150°C, more preferably between 80 and 130°C for 1 to 100 hrs, preferably 1 to 20 hrs.

When the reaction temperature exceeds 180°C, unfavorable side reactions, such as polymerization of the cyclic lactone (3), become marked.

The N-alkylcarbamylalkanol (4) obtained by this process may be used in the subsequent step as is. If desired, however, it may be purified by recrystallization from a solvent, such as hexane, methanol, ethanol, acetone and chloroform, to thereby obtain an N-alkylcarbamylalkanol having increased purity.

### step B'

This is a step in which the N-alkylcarbamylalkanol (4) obtained in the steps A' or D' is reacted with an alkylene oxide in the presence of an alkali or acid catalyst to thereby obtain the N-alkylcarbamylalkanol represented by the above general formula (5) (hereinafter referred to simply as "N-alkylcarbamylalkanol (5)").

Examples of the alkali catalysts include alkali metal hydroxides, alkali metal carbonates, organic amines, metal oxides and alkoxides. Among them, NaOH and KOH are preferred.

Examples of the acid catalysts include Lewis acids and metal oxides, such as boron trifluoride (BF₃), tin tetrachloride (SnCl₄), antimony pentachloride (SbCl₅), magnesium oxide (MgO) and aluminum oxide (Al₂O₃). Among them, boron trifluoride and magnesium oxide are preferred.

Examples of the alkylene oxides include ethylene oxide and propylene oxide. Ethylene oxide is preferred.

In this step, the N-alkylcarbamylalkanol (5) can be obtained by reacting the N-alkylcarbamylalkanol (4) with a desired amount of an alkylene oxide in the presence of an acid or alkali catalyst in an amount of 0.5 to 5 % by mol based on the molar number of the N-alkylcarbamylalkanol (4) as the starting compound at 120 to 180°C for 1 to 10 hrs.

When the reaction temperature exceeds 180°C, various unfavorable by-products that cause discoloration or smell, such as aldehydes and peroxides, are yielded.

After the completion of the reaction, an alkali, such as NaOH and KOH, may be added to the reaction mixture in order to neutralize the acid catalyst, or an acid, such as phosphoric acid, acetic acid and lactic acid, may be added to the reaction mixture in order to neutralize the alkali catalyst.
Alternatively or further, treatment of the reaction mixture with an acid or alkali adsorbent for purification may be effected to remove a salt.

### step B or C'

This is a step in which the N-alkylcarbamylalkanol (4) or (5) is reacted with a sulfating agent to thereby obtain a N-alkylcarbamylalkanol sulfate represented by the above general formula (1-1) or (2-1) (with the proviso that Ma is a hydrogen atom), and then, if necessary, neutralizing the N-alkylcarbamylalkanol sulfate thus obtained with a basic compound to thereby obtain a salt of N-alkylcarbamylalkanol sulfate represented by the above general formulae (1-1) or (2-1) with the proviso that Ma is not a hydrogen atom) or (1-2) or (2-2).

The N-alkylcarbamylalkanol sulfate or the salt thereof represented by the above general formula (1-1) or (2-1)" is referred to simply as "N-alkylcarbamylalkanol sulfate or the salt thereof (1-1) or (2-1)" and the salt of N-alkylcarbamylalkanol sulfate represented by the above general formula (1-2) or (2-2) is referred to simply as "the salt of N-alkylcarbamylalkanol sulfate (1-2) or (2-2)", hereinafter.

Examples of the sulfating agents include chlorosulfonic acid, sulfuric anhydride, fuming sulfuric acid, concentrated sulfuric acid and sulfamic acid. From the viewpoint of reaction yield, chlorosulfonic acid, sulfuric anhydride and sulfamic acid are preferred.

Examples of the basic compound include a hydroxide, a carbonate or a bicarbonate of an alkali metal or alkaline earth metal, ammonia, an alkanolamine having 2 to 9 carbon atoms in total, an alkylamine having 1 to 22 carbon atoms in total, an alkenylamine having 2 to 22 carbon atoms in total, a C₁-C₁₈ alkyl- or C₂-C₁₈ alkenylsubstituted pyridine and a basic amino acid. Specific examples thereof include inorganic alkalis, such as sodium hydroxide, potassium hydroxide, lithium hydroxide, barium hydroxide, sodium hydrogencarbonate, sodium carbonate and potassium carbonate, ammonia, monoethanolamine, diethanolamine, triethanolamine, methylamine, dimethylamine, trimethylamine, ethylamine, diethylamine, triethylamine, decylamine, dodecylamine, tetradecylamine, hexadecylamine, octadecylamine and basic amino acids. Among them, ammonia, sodium hydroxide, potassium hydroxide, monoethanolamine, diethanolamine and triethanolamine are preferred. In this step, the above basic compounds may be used in the form of an aqueous or alcohol solution thereof to neutralize the sulfate. The degree of neutralization may be set at an arbitrary level.

In this step, a sulfating agent is used in an amount of 1 to 1.5 times by mol that of the N-alkylcarbamylalkanol (4) or the N-alkylcarbamylalkanol (5). The reaction is effected in a solvent, such as dichloromethane, chloroform, 1,2-dichloroethane, dimethylformamide, dimethyl sulfoxide and dioxane, in an amount of 0 to 10 times by volume that of the N-alkylcarbamylalkanol (4) or the N-alkylcarbamylalkanol (5) at -50 to 80°C, preferably -30 to 50°C for 1 to 20 hrs. A sulfate (1-1) (with the proviso that Ma is a hydrogen atom) corresponding to the N-alkylcarbamylalkanol (4) or the N-alkylcarbamylalkanol (5) used can be obtained thereby.

The solvent to be used must be purified until there is scarcely any water or alcohol contained therein. The sulfation reaction can be conducted in the absence of any solvent. Without the solvent, however, the viscosity of the reaction mixture is extremely increased, so that countermeasures with respect to the equipment will be required.

Further, when the reaction temperature exceeds 80°C, unfavorable side reactions, such as cleavage of an amide group, would occur.

After the completion of the sulfation reaction, if desired, the solvent used in the sulfation reaction is distilled off. The distillation of the solvent can be conducted after the neutralization, which will be described hereinbelow.

Thereafter, the sulfate obtained by the above reaction is neutralized with a basic compound in an amount of 0.9 to 1.5 times by equivalent that of the N-alkylcarbamylalkanol (4) or the N-alkylcarbamylalkanol (5) in a solvent, such as water, ethanol and methanol, at -50 to 80°C for 0.1 to 1 hr to thereby obtain a salt of N-alkylcarbamylalkanol sulfate (1-1) (with the proviso that Ma is not a hydrogen atom) or (1-2).

By-products, such as N-alkylcarbamylalkanols, lactones and amines, are present in the reaction mixture obtained by the above neutralization reaction. Depending on use, the reaction mixture can be employed as it is. If desired, however, the reaction mixture is purified by recrystallization, column chromatography, electrodialysis, solvent extraction, etc., to thereby obtain a product having increased purity.

### step C or D'

This is a step in which the aliphatic amine (2) is reacted with an ω-hydroxycarboxylic acid or an ester thereof represented by the above general formula (6) (hereinafter referred to simply as "ω-hydroxycarboxylic acid or ester (6)"), preferably an ω-hydroxycarboxylic ester (6), to thereby obtain a N-alkylcarbamylalkanol (4).

Needless to say, this step includes a step in which the aliphatic amine (2) is reacted with an ω-hydroxycarboxylic acid represented by the general formula (6'): HO-R³CO₂H (6') (wherein R³ is defined above) to thereby obtain a N-alkylcarbamylalkanol (4).

Examples of the ω-hydroxycarboxylic acids or esters (6) include lactic acid, glycolic acid (1-hydroxyacetic acid), 2-hydroxypropionic acid, 3-hydroxybutyric acid, 4-hydroxypentanoic acid, 4-hydroxy-3-methylpentanoic acid and 5-hydroxyhexanoic acid, and methyl, ethyl, propyl and butyl esters thereof. From the viewpoints of foaming power and chemical stability of the surfactant to be obtained by this production process, desired examples of ω-hydroxycarboxylic acids include 3-hydroxybutyric acid, 4-hydroxypentanoic acid, 4-hydroxy-3-methylpentanoic acid and 5-hydroxyhexanoic acid. From the viewpoints of foaming power and chemical stability of the surfactant to be obtained by this production process, desired examples of ω-hydroxycarboxylic acid esters include methyl glycolate, ethyl glycolate, propyl glycolate, butyl glycolate, methyl 2-hydroxypropionate, methyl 3-hydroxybutyrate, methyl 4-hydroxypentanoate and methyl 5-hydroxyhexanoate. Among them, methyl glycolate, ethyl glycolate and methyl 5-hydroxyhexanoate are especially preferred.

The ω-hydroxycarboxylic acids or esters (6) can be produced by known processes, such as hydrolysis and treatment with an alcohol of the corresponding lactone. In the present invention, those produced by known processes may be used, or alternatively, commercial products may be used either as they are or after purification, such as recrystallization and distillation.

In this step, the aliphatic amine (2) and ω-hydroxycarboxylic acid or ester (6), in an amount of 1 to 2.0 times by mole that of the aliphatic amine (2), are subjected to reaction for removal of a water or alcohol molecule in the absence of any solvent, and if desired, in the presence of a basic catalyst, such as CH₃ONa, KOH and NaOH, at 40 to 200°C, preferably 60 to 150°C for 1 to 100 hrs, preferably 1 to 20 hrs, at a reduced pressure. The N-alkylcarbamylalkanol (4) can be obtained thereby.

In the reaction, the pressure is preferably reduced to 760 - 0.1 mmHg to effect dehydration.

When ω-hydroxycarboxylic acid is used, the aliphatic amine (2) and ω-hydroxycarboxylic acid in an amount of 1 to 2.0 times by mole that of the aliphatic amine (2) are subjected to reaction for removal of a water or alcohol molecule in the absence of any solvent at 100 to 250°C, preferably 150 to 200°C for 5 to 100 hrs at a reduced pressure.

The N-alkylcarbamylalkanol (4) thus obtained may be used as it is in the subsequent step. If desired, however, it may be purified by recrystallization from a solvent, such as hexane, methanol, ethanol, acetone and chloroform.

The N-alkylcarbamylalkanol sulfate or the salt thereof (1-1) or (2-1) or the salt of N-alkylcarbamylalkanol sulfate (1-2) or (2-2) according to the present invention is a surfactant ensuring not only excellent performance in detergency and foaming power but also high safety, so that it is suitable for use in various detergents such as a shampoo, a detergent for the body and a detergent for tableware.

The detergent composition of the present invention contains the N-alkylcarbamylalkanol sulfate or the salt thereof (1-1) or the salt of N-alkyl-carbamylalkanol sulfate (1-2) as an essential ingredient. The content thereof (the total content when two or more compounds are used) is preferably in the range of 1 to 70% by weight, still preferably in the range of 10 to 70% by weight based on the total weight of the composition.

The detergent composition of the present invention may also contain ingredients used in conventional detergents such as a shampoo, a detergent for the body and a detergent for the tableware, as long as the effect of the present invention is not adversely affected. Ingredients that may be used with the essential ingredient include silicone derivatives, cationic surfactants, water-soluble cationic polymers, anionic surfactants, nonionic surfactants, amphoteric surfactants, humectants, bactericides, emulsifiers and fragrances.

Examples of the silicone derivatives suitable for use in the present invention include the following silicone derivatives 1) to 9):
1) dimethylpolysiloxane

   (CH₃)₃SiO[(CH₃)₂SiO]ₘSi(CH₃)₃

   (m = 3 to 20,000)
2) methylphenylpolysiloxane 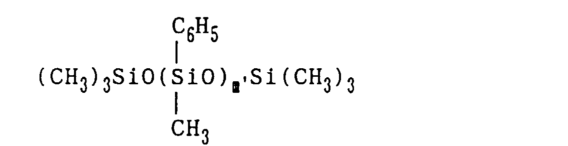 (m' = 1 to 20,000)
   or

   (CH₃)₃SiO[(CH₃)₂SiO]ₐ[(C₆H₅)₂SiO]_{b}Si(CH₃)₃

   (wherein a + b = 1 to 500)
3) polyether-modified silicone 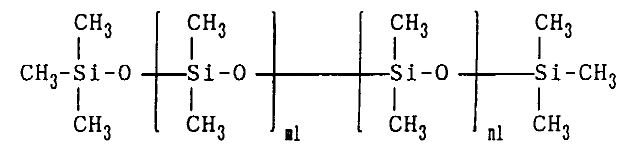 or 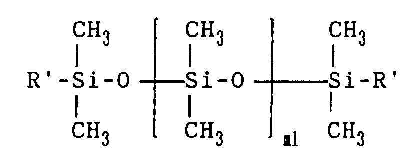 or 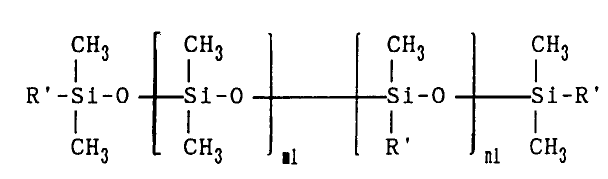 wherein
   - R':: 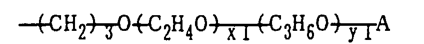
   - A:: alkyl group having 1 to 12 carbon atoms or hydrogen atom
   - x₁:: 0~50 y₁: 0~50 (x₁+y₁≧1)
   - m₁:: 1∼2000
   - n₁:: 1∼1000
4) epoxy-modified silicone 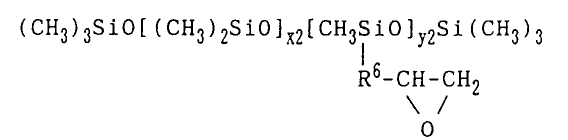 wherein x₂ is a number of 1 to 500, preferably 1 to 250, y₂ is a number of 1 to 50, preferably 1 to 30 and R⁶ represents an alkylene group having 1 to 3 carbon atoms
5) fluorinated silicone 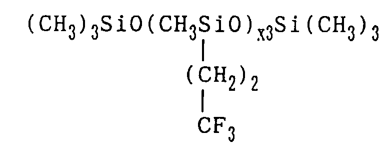 wherein x₃ is a number of 1 to 400, preferably 1 to 250
6) alcohol-modified silicone

   HO(CH₂)-R⁷-[(CH₃)₂SiO]ₓ₄(CH₃)₂SiR⁷-CH₂OH

   or 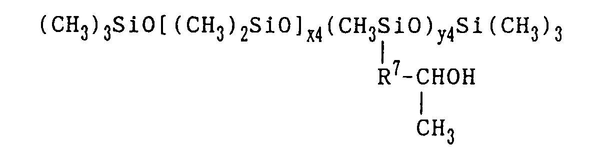 wherein x₄ and y₄ are each a number of 1 to 500, preferably 1 to 200 and R⁷ represents C_{n'}H_{2n'} (wherein n' = 0 to 4)
7) alkyl-modified silicone 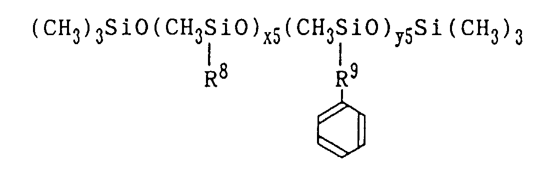 or 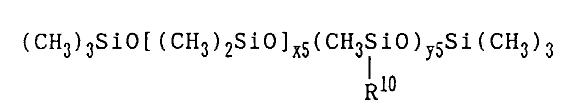 wherein x₅ and y₅ are each a number of 1 to 500, preferably 1 to 200, R⁸ represents an alkyl group having 2 to 18 carbon atoms, R⁹ represents C_{n'}H_{2n'} (wherein n' = 0 to 4), and R¹⁰ represents an alkyl group having 10 to 24 carbon atoms
8) alkoxy-modified silicone 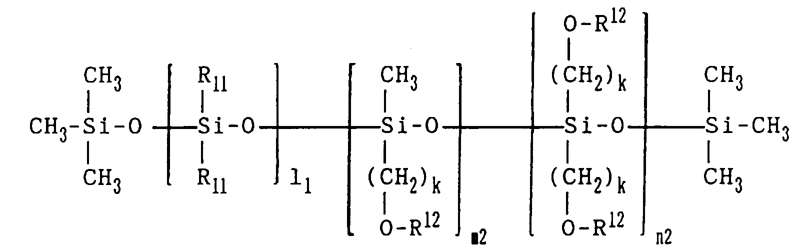 wherein R¹¹ represents a methyl group or a phenyl group, l₁ is a number of 1 to 3000, m₂ and n₂ are numbers satisfying the relation m₂ + n₂ = 1 to 500 , R¹² represents an alkyl group having 1 to 28 carbon atoms, preferably 12 to 22 carbon atoms, and k is an integer of 0 to 6, and
9) amine-modified silicone 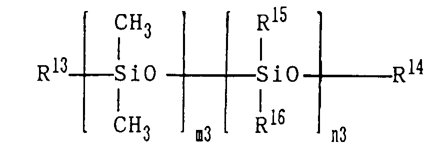 or 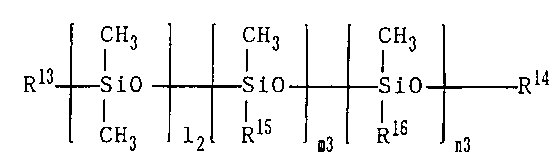 wherein R¹³ represents a methyl group or a hydroxyl group; R¹⁴ represents a methyl group or a hydrogen atom; R¹⁵ represents an aminoalkyl group represented by the formula: 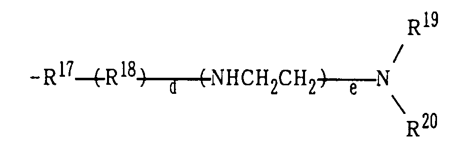 or 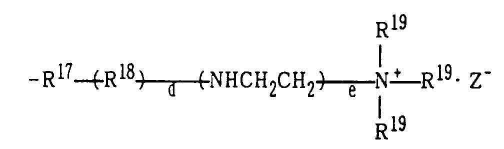 wherein R¹⁷ represents a divalent hydrocarbon group, R¹⁸ represents the group of -OCH₂CH₂-, 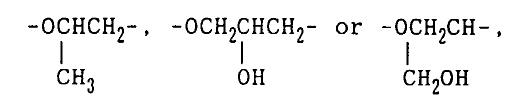 each of R¹⁹ and R²⁰ represents a hydrogen atom or a monovalent hydrocarbon group, each of d and e is a number of 0 to 6, and Z⁻ represents a halide ion or an organic anion;
   R¹⁶ represents a hydroxy group, a hydroxyalkyl group, an oxyalkylene group [i.e., a group represented by -(OA)-OH (wherein OA represents an oxyalkylene group)] or a polyoxyalkylene group [i.e., a group represented by -(OA)ₚ-OH (wherein OA represents an oxyalkylene group and p is an average addition mole number of alkylene oxide)]; and each of l₂, m₃ and n₃ is a number depending on the molecular weight.

Among them, especially preferred amine-modified silicones are those represented by the following general formula: 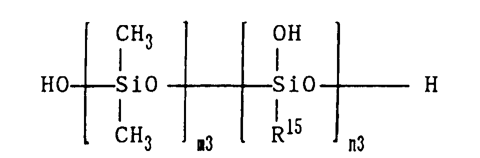 wherein R¹⁵, m₃ and n₃ are defined above.

Among them, representative are those represented by the following formula: 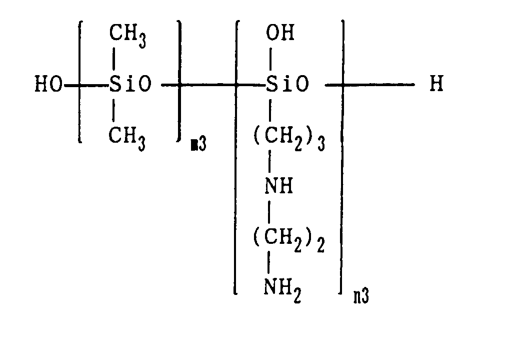 wherein m₃ and n₃ are defined above.

As the amine-modified silicone, those represented by the above formula and having an average molecular weight of about 3,000 to 100,000, and which are named Amodimethicone and described in the dictionary of CTFA (U.S., Cosmetic Ingredient Dictionary), 3rd edition, are preferred.

It is preferred that the above amine-modified silicone be used in the form of a water-base emulsion. Such a water-base emulsion can be obtained, for example, by performing an emulsion polymerization of a cyclic diorganopolysilocane and an organodialkoxysilane having an aminoalkyl group, and a hydroxy group, a hydroxyalkyl group, an oxyalkylene group or a polyoxyalkylene group, in the presence of a surfactant based on a quaternary ammonium salt together with water, in accordance with the process described in U.S. Patent No. 4,228,054 (published on Oct. 14, 1980; assignee: Toray Silicone Co., Ltd.).

When the above amine-modified silicone is used in the form of a water-base emulsion, the content of the amine-modified silicone in the emulsion is generally in the range of 20 to 60% by weight, preferably 30 to 50% by weight.

Preferred water-base amine-modified silicone emulsions are commercially available, which include, for example, SM 8702C (produced by Toray Silicone Co., Ltd.) and DC 929 (produced by Dow Corning Corporation).

These silicone derivatives may be used individually or in combination of two or more, and incorporated in the detergent composition of the present invention in an amount of preferably 0.01 to 3% by weight, still more preferably 0.1 to 0.8% by weight.

The silicone derivatives 1) to 9) described above may be used as such or in the form of a silicone emulsion wherein a silicone derivative is dispersed in an aqueous medium with at least one surfactant, as an emulsifier, selected from the group consisting of a nonionic surfactant, an anionic surfactant, a cationic surfactant and an amphoteric surfactant. The silicone emulsion may be obtained by stirring and mixing the mixture comprising a silicone derivative and an aqueous medium or by an emulsion polymerization process of the silicone derivative. Examples of silicone emulsions which are put on the market include KM 880, KM 883, KM 884, KM 885, KM 886 and KM 887 (mfd. by Shin-Etsu Chemical Industries Co. Ltd.), TEX 100, TSW 831, TEX 150, TEX 154, TEX 170, TEX 152 and TEX 172 (mfd. by Toshiba Silicone Co. Ltd.) and BY22-007, BY22-029, BY22-019, BY22-034, BY22-020, BY22-009, BY22-008 and SM 5571 (mfd. by Toray Dow Corning Silicone Co. Ltd.).

The cationic surfactants for use in the present invention include, for example, quaternary ammonium salts represented by the following general formulae (7) and (8): 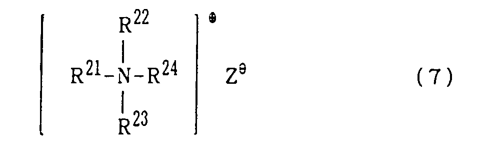 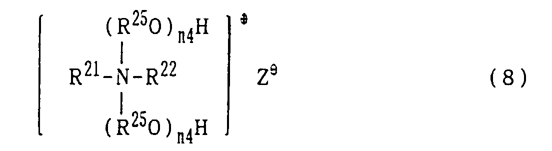 wherein at least one of R²¹, R²², R²³ and R²⁴ represents an alkyl or alkenyl group that may be substituted with an alkoxy, alkenyloxy, alkanoylamino or alkenoylamino group, having 8 to 28 carbon atoms in total, and the remainings of R²¹, R²², R²³ and R²⁴ represent a benzyl group, an alkyl or hydroxyalkyl group having 1 to 5 carbon atoms; R²⁵ represents an alkylene group having 2 or 3 carbon atoms; Z represents a halide ion or an organic anion; and n₄ is a number of 1 to 20. Among the above cationic surfactants, quaternary ammonium salts represented by the general formula (7) are preferred. Further, among the quaternary ammonium salts represented by the general formula (7), branched quaternary ammonium salts represented by the following general formulae (9) to (11) are particularly preferred. 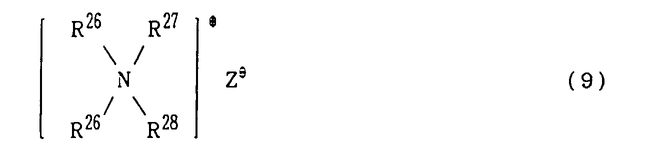 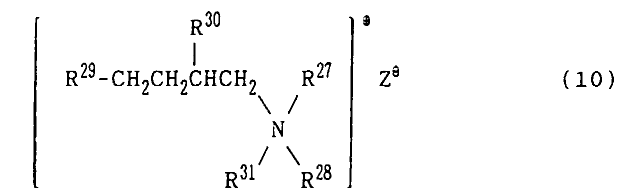 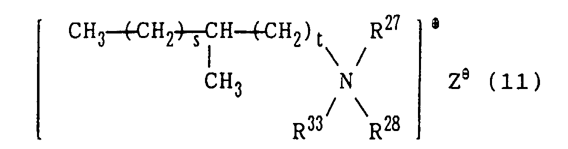 wherein R²⁶ is a branched alkyl group represented by the formula (a): 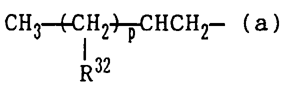 (wherein R³² represents a methyl group or an ethyl group, and p is an integer selected so as to allow the total number of carbon atoms in the alkyl group to be 8 - 16, or a linear alkyl group represented by the formula (b): 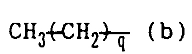 (wherein q is an integer of 7 to 15); each of R²⁷ and R²⁸ represents a benzyl group or an alkyl or hydroxyalkyl group having 1 to 3 carbon atoms; each of R²⁹ and R³⁰ represents an alkyl group having 2 to 12 carbon atoms; R³¹ represents a group of 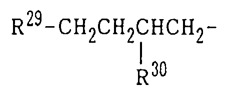 or an alkyl group having 1 to 3 carbon atoms; R³³ represents a group of 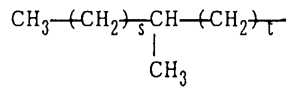 or an alkyl group having 1 to 3 carbon atoms; s and t are integers of 2 to 14 and 3 to 11, respectively, and the sum of s and t is 9 to 21; and Z represents a halide ion or an organic anion.

The surfactant, i.e., the branched quaternary ammonium salt, represented by the general formula (9) has a branching ratio of R²⁶ [formula (a)/formula (a) + formula (b)] of 10 to 100%, and those having a branching ratio of 10 to 50% are particularly preferred.

The surfactant represented by the general formula (9) is synthesized, for example, from an oxo alcohol having 8 to 16 carbon atoms as a starting compound. Examples thereof include a dialkyldimethylammonium salt, a dialkylmethylhydroxyethylammonium salt and a dialkylmethylbenzylammonium salt, each having an alkyl group derived from an oxo alcohol.

Although one alkyl group of R²⁶ has 8 to 16 carbon atoms in the general formula (9), those wherein a given distribution, especially the following distribution, is present with respect to the carbon atom number of one alkyl group of R²⁶, among surfactants represented by the general formula (9), are preferably used in the present invention:
- C₈~C₁₁:: 5% or less
- C₁₂:: 10∼35%
- C₁₃:: 15∼40%
- C₁₄:: 20∼45%
- C₁₅:: 5∼30%
- C₁₆:: 5% or less

Specific examples of the above surfactant, i.e., the branched quaternary ammonium salt, include a dialkyldimethylammonium chloride having an alkyl group of 8 to 16 carbon atoms and a branching ratio of 10 to 50%.

The branched quaternary ammonium salt represented by the general formula (10) is generally synthesized from a Guerbet alcohol 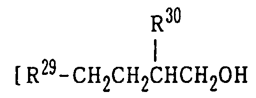 (wherein R²⁹ and R³⁰ are each defined above)] having 8 to 28 carbon atoms, as a starting compound. Preferred examples of the branched quaternary ammonium salts include alkyltrimethylammonium salts, alkyldimethylbenzylammonium salts, dialkyldimethylammonium salts, dialkylmethylhydroxyethylammorium salts and dialkylmethylbenzylammonium salts, each having an alkyl group derived from a Guerbet alcohol. Among them, especially preferred examples include 2-decyltetradecyltrimethylammonium chloride, 2-dodecylhexadecyltrimethylammonium chloride, di-2-hexyldecyldimethylammonium chloride and di-2-octyldodecyidimethylammonium chloride.

Preferred examples of the methyl-branched quaternary ammonium salts represented by the general formula (11) are those having s and t values totaling 15.

Specific examples of Z as a counter ion for each of the quaternary ammonium salts represented by the general formulae (7), (8), (9), (10) and (11) include halide (such as chloride, iodide and bromide) ions; and organic anions, such as methosulfate, ethosulfate, methophospahte and ethophosphate.

The above cationic surfactants may be used either individually or in combination of two or more, and incorporated in the detergent composition of the present invention preferably in an amount of 0.05 to 10% by weight.

The water-soluble cationic polymer for use in the present invention is a water-soluble polymer either comprising a polymer chain having a amino group or an ammonium group bonded thereto or comprising at least a dimethyldiallylammonium halide as a structural unit. Examples thereof include cationized cellulose derivatives, cationic starches, cationized guar gum derivatives, copolymers of quaternary diallylammonium salt and acrylamide, and quaternary polyvinylpyrrolidone derivatives.

As the cationized cellulose derivatives, for example, those represented by the following general formula (12) are preferred:

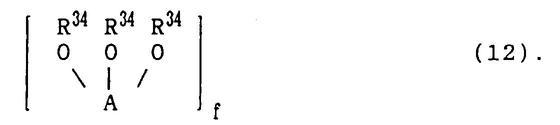

In the formula (12), A represents an anhydroglucose unit residue, f is a number of 50 to 20000, and R³⁴ is a substituent represented by the following general formula (13):

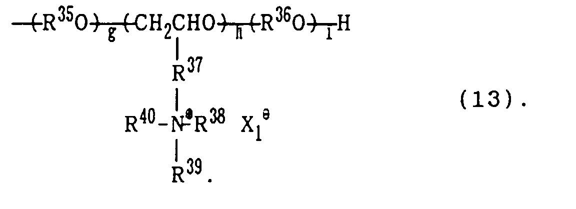

In the formula (13), R³⁵ and R³⁶ may be the same or different from each other and each represents an alkylene group having 2 or 3 carbon atoms; g is a number of 0 to 10; h is a number of 0 to 3; i is a number of 0 to 10; R³⁷ represents an alkylene or hydroxyalkylene group having 1 to 3 carbon atoms; R³⁸, R³⁹ and R⁴⁰ may be the same or different from one another and each represents an alkyl, aryl or aralkyl group having 1 to 10 carbon atoms, or may form a heterocycle together with the nitrogen atom in the formula (13); and X₁⁻ represents an anion, e.g., chloride ion, bromide ion, iodide ion, sulfonate ion, methylsulfate ion or nitrate ion.

Similar to the cationized cellulose derivative represented by the formula (12), those having a divalent anion such as sulfate ion, and others having a trivalent anion such as phosphate ion, are cited. In such cationized cellulose derivatives, the valence of anion and the valence of cation are identical, as well.

As the cationized cellulose derivative used in the present invention, those represented by the formula (12) which have the degree of cationization, that is, the average value of h per anhydroglucose unit, of 0.01 to 1 are preferable and those represented by the formula (12) which have the degree of cationization of 0.02 to 0.5 are still more preferable. Such cationized cellulose derivatives have a sum of g and i (average value) of 1 to 3. A degree of cationization of less than 0.01 is not satisfactory. Although those having a degree of cationization greater than 1 may be used, the synthesis thereof are disadvantageous from the viewpoint of the reaction yield. It is preferred that the average molecular weight of the cationized cellulose derivative to be employed in the present invention be in the range of about 100,000 to 3,000,000.

As the cationic starches, those represented by the following general formula (14) are preferred:

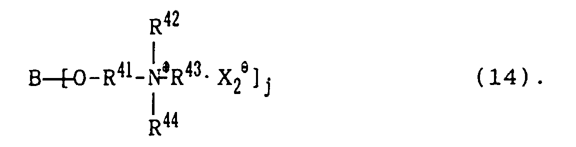

In the formula (14), B represents a starch residue; R⁴¹ represents an alkylene group or a hydroxyalkylene group; R⁴², R⁴³ and R⁴⁴ may be the same or different from one another and each represents an alkyl, aryl or aralkyl group having 1 to 10 carbon atoms, or may form a heterocycle together with the nitrogen atom in the formula; X₂⁻ represents an anion, e.g., chloride ion, bromide ion, iodide ion, sulfonate ion, methylsulfate ion or nitrate ion; and j is a positive number.

Similar to the cationic starch represented by the formula (14), those having a divalent anion such as sulfate ion, and others having a trivalent anion such as phosphate ion, are cited. In such cationic starches, the valence of anion and the valence of cation are identical.

As the cationic starch to be used in the present invention, those represented by the formula (14) which have the degree of cationization of 0.01 to 1, that is, those in which 0.01 to 1 cation group is introduced per anhydroglucose unit, are preferred, and those in which 0.02 to 0.5 cation group is introduced per anhydroglucose unit are still more preferred. A degree of cationization less than 0.01 is not satisfactory. Although those having a degree of cationization greater than 1 may be used, the synthesis thereof are disadvantageous from the viewpoint of the reaction yield.

As the cationized guar gum derivatives, those represented by the following general formula (15) are preferred:

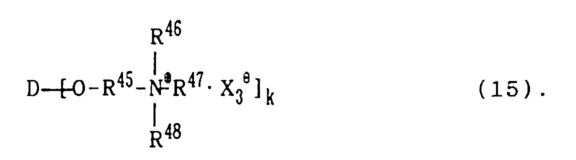

In the formula (15), D represents a guar gum residue; R⁴⁵ represents an alkylene or hydroxyalkylene group; R⁴⁶, R⁴⁷ and R⁴⁸ may be the same or different from one another and each represents an alkyl, aryl or aralkyl group having 1 to 10 carbon atoms, or may form a heterocycle together with the nitrogen atom in the formula; X₃⁻ represents an anion, e.g., chloride ion, bromide ion, iodide ion, sulfonate ion, methylsulfate ion or nitrate ion; and k is a positive number.

Similar to the cationized guar gum derivative represented by the formula (15), those having a divalent anion such as sulfate ion, and others having a trivalent anion such as phosphate ion, are cited. In such cationized guar gum derivatives, the valence of anion and the valence of cation are identical, as well.

As the cationized guar gum derivative to be used in the present invention, those represented by the formula (15) which have the degree of cationization of 0.01 to 1, that is, those in which 0.01 to 1 cation group is introduced per glucose unit, are preferred, and those in which 0.02 to 0.5 cation group is introduced per glucose unit are still more preferred. This type of cationic polymer is described in, for example, U.S. Patent Nos. 4,298,494 (published on Nov. 3, 1981; assignee: UNILEVER NV), 5,037,818 (published on Aug. 6, 1991; assignee: UNILEVER NV, CHESEBROUGH PONDS INC) and 4,364,837 (published on Dec. 21, 1982; assignee: LEVER BROTHERS CO.), and is commercially available under the trademark of Jaguar (product by Celanese Stein Hall).

As the cationic copolymers of quaternary diallylammonium salt and acrylamide, those represented by the following general formulae (16) and (17) are preferred: 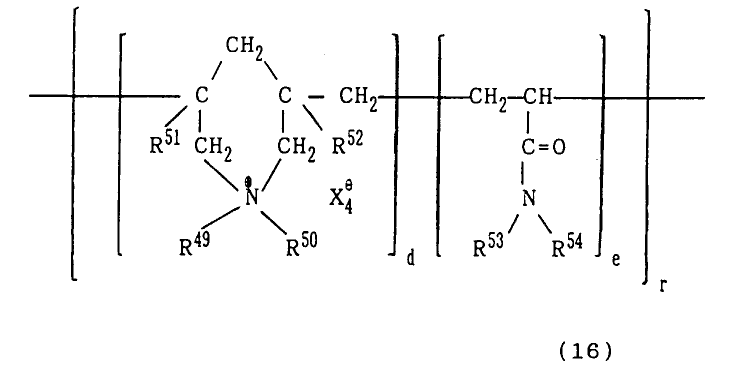 and

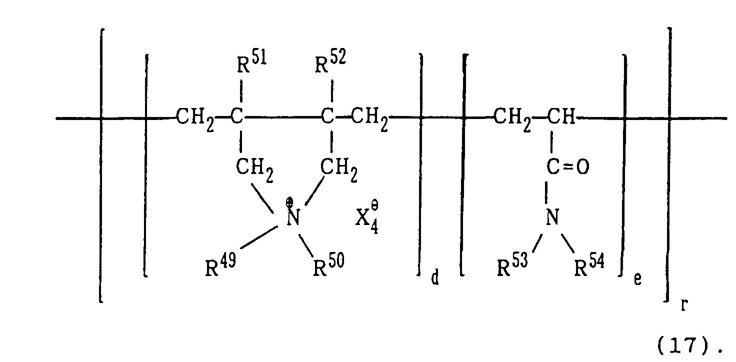

In the formulae (16) and (17), R⁴⁹ and R⁵⁰ may be the same or different from each other and each represents a hydrogen atom, an alkyl group having 1 to 18 carbon atoms, a phenyl group, an aryl group, a hydroxyalkyl group, a carbamylalkyl group, a cyanoalkyl group, an alkoxyalkyl group or a carboalkoxyalkyl; R⁵¹, R⁵², R⁵³ and R⁵⁴ may be the same or different from one another and each represents a hydrogen atom, a lower alkyl group having 1 to 3 carbon atoms or a phenyl group; X₄⁻ represents an anion, e.g., chloride ion, bromide ion, iodide ion, sulfonate ion, methylsulfate ion or nitrate ion; d is a number of 1 to 50; e is a number of 0 to 50; and r is a number of 150 to 8000.

Similar to the copolymer of quaternary diallylammonium salt and acrylamide represented by the formulae (16) or (17), those having a divalent anion such as sulfate ion, and others having a trivalent anion such as phosphate ion, are cited. In such copolymers of quaternary diallylammonium salt and acrylamide, the valence of anion and the valence of cation are identical, as well.

As the copolymer of quaternary diallylammonium salt and acrylamide to be used in the present invention, those having an average molecular weight of about 30,000 to 2,000,000 are preferred, and those having an average molecular weight of 100,000 to 1,000,000 are still more preferred.

As the quaternary polyvinylpyrrolidone derivatives, those represented by the following formula (18) are preferred:

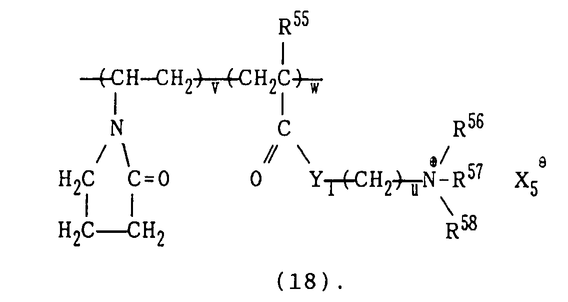

In the formulae (18), R⁵⁵ represents a hydrogen atom or an alkyl group having 1 to 3 carbon atoms; R⁵⁶, R⁵⁷ and R⁵⁸ may be the same or different from one another and each represents a hydrogen atom or an alkyl, hydroxyalkyl, carbamylalkyl, cyanoalkyl, alkoxyalkyl or carboalkoxyalkyl group having 1 to 4 carbon atoms; Y₁ represents an oxygen atom or an NH group forming an amide bond; X₅⁻ represents an anion, e.g., chloride ion, bromide ion, iodide ion, sulfonate ion, alkyl sulfate ion having an alkyl group of 1 to 4 carbon atoms or nitrate ion; u is an integer of 1 to 10; and v + w is a number of 20 to 8000.

Similar to the quaternary polyvinylpyrrolidone derivative represented by the formula (18), those having a divalent anion such as sulfate ion, and others having a trivalent anion such as phosphate ion, are cited. In such quaternary polyvinylpyrrolidone derivatives, the valence of anion and the valence of cation are identical, as well.

As the quaternary polyvinylpyrrolidone derivative to be used in the present invention, those having an average molecular weight of 10,000 to 2,000,000 are preferred, and those having an average molecular weight of 50,000 to 1,500,000 are still more preferred.

The above cationic polymers may be used either individually or in combination of two or more, and are incorporated into the detergent composition of the present invention in an amount of preferably 0.01 to 3% by weight, still more preferably 0.1 to 0.8% by weight.

Examples of the anionic surfactants for use in the present invention include alkyl sulfates, alkyl ether sulfates, alkyl sulfonates and sulfosuccinates.

Examples of the nonionic surfactants for use in the present invention include alkyl glycosides represented by the following formula (19), amine oxides, oxyalkylene alkyl ethers, monoglycerides and mono- and dialkanolamides: 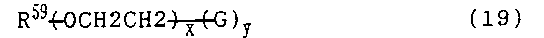 wherein R⁵⁹ represents a linear or branched, alkyl or alkenyl group having 8 to 18 carbon atoms, or an alkylphenyl group having an alkyl group of 8 to 18 carbon atoms; G represents a reducing sugar residue having 5 to 6 carbon atoms; x is a number of 0 to 20; and y is a number of 1 to 10.

Examples of the amphoteric surfactants for use in the present invention include (long-chain alkyl)dimethylcarboxy-methyl betaines.

Examples of the humectants for use in the present invention include glycerol, propylene glycol and ethylene glycol.

The N-alkylcarbamylalkanol sulfates and salts thereof according to the present invention ensures not only low irritancy to the skin but also excellent foaming power and detergency. Therefore, the detergent composition of the present invention containing the N-alkylcarbamylalkanol sulfate and/or the salt thereof is useful as detergents for hair, skin, tableware, etc.

### Examples

Hereinbelow, the present invention will be described in greater detail with reference to the following Examples, which should not be construed as limiting the scope of the invention.

### Example 1

### Synthesis of sodium salt of N-dodecyl-1-sulfoxyacetamide

n-C₁₂H₂₅NHCOCH₂OSO₃Na

### (1) Synthesis of N-dodecylglycolamide

181.4 g (1.0 mol) of dodecylamine, 104.1 g (1.0 mol) of ethyl glycolate and 7.5 g (40 mmol) of a methanol solution containing 28% sodium methylate were fed into a 500-ml four-necked flask equipped with a stirrer and a thermometer. The obtained mixture was stirred under a stream of nitrogen gas, and the temperature thereof was elevated to 100°C. At this temperature, the reaction mixture was stirred for 2 hr while distilling off formed methanol. The obtained reaction mixture was dissolved in 500 ml of ether at room temperature. The ether solution thus obtained was transferred to a separatory funnel, and washed with water. The ether phase was separated, and concentrated. The solid remaining after concentration was dried in a reduced pressure to thereby obtain 182.3 g of white powdery N-dodecylglycolamide (yield: 74.9%). The hydroxyl value of this N-dodecylglycolamide was 229.4 (calculated value: 230.5).

The infrared absorption spectrum data and ¹H-NMR spectrum data thereof were as follows:

### Infrared aborption spectrum (KBr tablet method)

3334 (O-H stretching), 3262 (N-H stretching), 2920, 2854 (C-H stretching), 1638 (C=0 stretching) cm⁻¹

### ¹H-NMR spectrum (δ, ppm) in CDCl₃

a: 0.89 ppm (t, 3H)
b: 1.30 ppm (m, 18H)
c: 1.55 ppm (m, 2H)
d: 3.27 ppm (q, 2H)
e: 7.02 ppm (b, 1H)
f: 4.02 ppm (s, 2H)
g: 5.10 ppm (b, 1H)

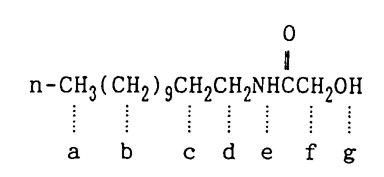

### (2) Synthesis of sodium salt of N-dodecyl-1-sulfoxyacetamide

35.5 g (146 mmol) of the N-dodecylglycolamide synthesized in the above step (1) was put in a 500-ml four-necked flask equipped with a stirrer, a dropping funnel, a condenser tube and a thermometer. Next, 350 ml of chloroform was put in the flask to thereby dissolve the N-dodecylglycolamide in the chloroform. While stirring the obtained chloroform solution at room temperature under a stream of nitrogen gas, 17.9 g (153 mmol) of chlorosulfonic acid was dropwise added thereto over a period of about 30 min. During the period, the flask was sometimes cooled in a water bath to prevent the reaction temperature from exceeding 40°C. After the completion of the dropwise addition, the mixture thus obtained was stirred at 30°C for 2 hrs. The reaction mixture thus obtained was poured into 500 ml of an ice water/n-butanol mixture containing 150 g of ice water. The obtained mixture was transferred to a separatory funnel, the funnel was vigorously shaked, and then an organic phase was separated. This organic phase was neutralized with a 10% aqueous sodium hydroxide solution, followed by solvent removal by distillation under reduced pressure and drying of the residue. Thus, 39.8 g of white powdery sodium salt of N-dodecyl-1-sulfoxyacetamide was obtained (yield: 78.9%). The infrared absorption spectrum data, ¹H-NMR spectrum data and mass spectrum data thereof were as follows:

### Infrared absorption spectrum (KBr tablet method)

3346 (N-H stretching), 2926, 2854 (C-H stretching), 1659 (C=0 stretching), 1251, 1070 (S=0 stretching) cm⁻¹

### ¹H-NMR spectrum (δ, ppm) in CDCl₃

a: 0.57 ppm (t, 3H)
b: 0.97 ppm (m, 18H)
c: 1.21 ppm (m, 2H)
d: 2.90 ppm (t, 2H)
e: 4.15 ppm (s, 2H)

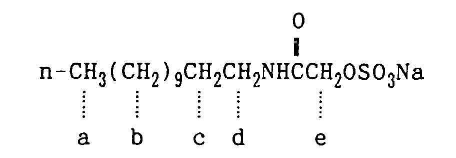

### Mass spectrum (Fab ionization method)

- 368:: (M+Na)⁺
- 266:: (M+H-SO₃)⁺
- 226:: (M-OSO₃Na)⁺

### Example 2

### Synthesis of ammonium salt of N-dodecyl-1-sulfoxyacetamide

n-C₁₂H₂₅NHCOCH₂OSO₃NH₄

35.5 g (146 mmol) of the N-dodecylglycolamide synthesized in the step (1) of above Example 1 was put in a 1-ℓ four-necked flask equipped with a stirrer, a dropping funnel, a condenser tube and a thermometer. Next, 500 ml of chloroform was put in the flask to thereby dissolve the N-dodecylglycolamide in the chloroform. While stirring the obtained chloroform solution at room temperature under a stream of nitrogen gas, 17.9 g (153 mmol) of chlorosulfonic acid was dropwise added thereto over a period of about 40 min. During the period, the flask was sometimes cooled in a water bath to prevent the reaction temperature from exceeding 40°C. After the completion of the dropwise addition, the mixture thus obtained was stirred at 25 to 30°C for 2 hrs. The reaction mixture thus obtained was poured into 600 ml of an ice water/n-butanol mixture containing 300 g of ice water. The obtained mixture was transferred to a separatory funnel, the funnel was vigorously shaked, and then an organic phase was separated. This organic phase was neutralized with 10% aqueous ammonia, followed by solvent removal by distillation under reduced pressure and drying the residue. Thus, 41.4 g of white powdery ammonium salt of N-dodecyl-1-sulfoxyacetamide was obtained (yield: 83.3%). The anionic surfactant purity thereof was found to be 97.7% by titration according to the Epton method. The infrared absorption spectrum data thereof were as follows:

### Infrared absorption spectrum (KBr tablet method)

3340 (N-H stretching), 2925, 2855 (C-H stretching), 1655 (C=0 stretching), 1245, 1071 (S=0 stretching) cm⁻¹

### Example 3

### Synthesis of sodium salt of N-dodecyl-N-methyl-1-sulfoxyacetamide

n-C₁₂H₂₅N(CH₃)COCH₂OSO₃Na

42.7 g (214 mmol) of dodecylmethylamine, 22.5 g (216 mmol) of ethyl glycolate and 2.1 g (0.7 mmol) of a methanol solution containing 28% sodium methylate were fed into a 200-ml four-necked flask equipped with a stirrer, a condenser tube and a thermometer. The obtained mixture was stirred under a stream of nitrogen gas, and the temperature thereof was elevated to 80°C. At this temperature, the mixture was stirred for 3 hrs while distilling off formed methanol. The temperature of the obtained reaction mixture was brought to room temperature, and then the reaction mixture was dissolved in 300 ml of ether. The ether solution thus obtained was transferred to a separatory funnel, and washed with water. The ether phase was separated, and concentrated. The liquid remaining after concentration was removed by distillation under reduced pressure and the residue was dried to thereby obtain N-dodecyl-N-methylglycolamide.

This amide was put in a 500 ml four-necked flask equipped with a stirrer, a dropping funnel, a condenser tube and a thermometer. Next, 300 ml of chloroform was put in the flask to thereby dissolve the N-dodecyl-N-methylglycolamide in the chloroform. While stirring the obtained chloroform solution at room temperature under a stream of nitrogen gas, 26.2 g (225 mmol) of chlorosulfonic acid was dropwise added thereto over a period of about 30 min. During the period, the flask was sometimes cooled in a water bath to prevent the reaction temperature from exceeding 30°C. After the completion of the dropwise addition, the obtained mixture was stirred at 25 to 30°C for 1 hr. The obtained reaction mixture was poured into 400 ml of an ice water/n-butanol mixture containing 200 g of ice water. The obtained mixture was transferred to a separatory funnel, the funnel was vigorously shaked, and then an organic phase was separated. This organic phase was neutralized with a 10% aqueous sodium hydroxide solution, followed by solvent removal by distillation under reduced pressure and drying the residue. Thus, 34.0 g of white powdery sodium salt of N-dodecyl- N-methyl-1-sulfoxyacetamide was obtained (yield: 44.1%). The anionic surfactant purity thereof was found to be 99.4% by titration according to the Epton method. The infrared absorption spectrum data, ¹H-NMR spectrum data and mass spectrum data thereof were as follows:

### Infrared absorption spectrum (KBr tablet method)

2926, 2854 (C-H stretching), 1655, 1647 (C=0 stretching), 1263, 1224, 1035 (S=0 stretching) cm⁻¹

### ¹H-NMR spectrum (δ, ppm) in CDCl₃

a: 0.59 ppm (t, 3H)
b: 1.00 ppm (m, 18H)
c: 1.22 ppm (m, 2H)
d: 3.01 ppm (m, 2H)
e: 2.60 ppm (s, 1.4H), 2.69 (s, 1.6H)
f: 4.39 ppm (s, 2H)

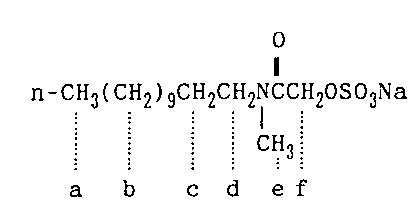

### Mass spectrum (Fab ionization method)

- 382:: (M+Na)⁺
- 280:: (M+H-SO₃)⁺
- 240:: (M-OSO₃Na)⁺

### Example 4

### Synthesis of sodium salt of N-dodecyl-5-sulfoxyhexanamide

### (1) Synthesis of N-dodecyl-5-hydroxyhexanamide

384.20 g (2.0727 mol) of dodecylamine was fed into a 2-ℓ four-necked flask equipped with a stirrer, a dropping funnel and a thermometer, and heated to 90°C. Then, 236.58 g (2.0727 mol) of ε-caprolactone was dropwise added to the dodecylamine over a period of about 0.5 hr. During this period, the temperature of the reaction system was maintained at 90 to 100°C. The reaction mixture was further stirred at 100°C for 5 hrs. The obtained crude product having a temperature of its melting point or above was dropwise added to 2.1 ℓ of hexane to crystallize. Thus, 509.53 g of N-dodecyl-5-hydroxyhexanamide was obtained (yield: 82%). The hydroxyl value thereof was 182.0 (calculated value: 187.33) and the infrared absorption spectrum data thereof were as follows:

### Infrared absorption spectrum (KBr tablet method)

3316 (O-H stretching), 2926, 2854 (C-H stretching), 1635 (C=0 stretching) cm⁻¹

### (2) Synthesis of sodium salt of N-dodecyl-5-sulfoxyhexanamide

460.26 g (1.5368 mol) of the N-dodecyl-5-hydroxyhexanamide obtained in the above step (1) was put in a 5-ℓ round-bottomed flask equipped with a stirrer, a dropping funnel, a condenser tube and a thermometer. Next, 2 ℓ of chloroform was put in the flask to thereby dissolve the N-dodecyl-5-hydroxyhexanamide in the chloroform. While stirring the obtained chloroform solution at room temperature under a stream of nitrogen gas, 189 g (1.62 mol) of chlorosulfonic acid was dropwise added thereto over a period of about 1 hr. During the period, the temperature of the reaction system was maintained at 10 to 20°C. Thereafter, the obtained mixture was further stirred at room temperature for 1 hr. The obtained reaction mixture was poured into 1 ℓ of an ice water/n-butanol mixture containing 300 ml of ice water. The obtained mixture was stirred for about 10 min, and then transferred to a separatory funnel. An organic phase was separated. This organic phase was neutralized with a 30% aqueous sodium hydroxide solution to adjust the pH thereof to 7. Chloroform and butanol were distilled off from the organic phase under reduced pressure. The remaining solid was washed with 2 ℓ of acetone to obtain 575.06 g of sodium salt of N-dodecyl-5-sulfoxyhexanamide (yield: 93%). The infrared absorption spectrum data, ¹H-NMR spectrum data and mass spectrum data of the obtained compound were as follows:

### Infrared absorption spectrum (KBr tablet method)

3338 (N-H stretching), 2925, 2855 (C-H stretching), 1640 (C=0 stretching), 1200, 1069 (S=0 stretching) cm⁻¹

### ¹H-NMR spectrum (δ, ppm) in D₂O

a: 0.93 ppm (t, 3H)
b: 1.40 ppm (b, 18H)
c: 1.50 ppm (m, 2H)
d: 3.18 ppm (t, 2H)
e: 2.30 ppm (t, 2H)
f: 1.68 ppm (m, 6H)
g: 4.09 ppm (t, 2H)

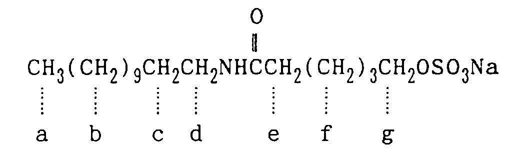

### Mass spectrum (Fab ionization method)

- 424:: (M+Na)⁺
- 322:: (M+H-SO₃)⁺
- 282:: (M-OSO₃Na)⁺

### Example 5

### Synthesis of ammonium salt of N-dodecyl-5-sulfoxyhexanamide

600.0 g (2.0 mol) of the N-dodecyl-5-hydroxyhexanamide synthesized in step (1) of the above Example 4 was put in a 5-ℓ four-necked flask equipped with a stirrer, a dropping funnel, a condenser tube and a thermometer. Next, 2 ℓ of chloroform was put in the flask to thereby dissolve the N-dodecyl-5-hydroxyhexanamide in the chloroform. While stirring the obtained chloroform solution at room temperature under a stream of nitrogen gas, 270.4 g (2.5 mol) of chlorosulfonic acid was dropwise added thereto over a period of about 1 hr. During the period, the flask was sometimes cooled in a water bath to prevent the reaction temperature from exceeding 40°C. After the completion of the dropwise addition, the obtained mixture was stirred at 25 to 30°C for 2 hrs. The obtained reaction mixture was poured into 2 ℓ of an ice water/n-butanol mixture containing 1 kg of ice water. The obtained mixture was transferred to a separatory funnel, the funnel was vigorously shaked, and then an organic phase was separated. This organic phase was neutralized with 10% aqueous ammonia, followed by solvent removal by distillation under reduced pressure and drying the residue. Thus, 713.2 g of white powdery ammonium salt of N-dodecyl-5-sulfoxyhexanamide was obtained (yield: 90.0%). The anionic surfactant purity thereof was found to be 99.9% by titration according to the Epton method. The infrared absorption spectrum data thereof were as follows:

### Infrared absorption spectrum (KBr tablet method)

3334, 3214 (N-H stretching), 2926, 2854 (C-H stretching), 1641 (C=0 stretching), 1209, 1065, 1044 (S=0 stretching) cm⁻¹

### Example 6

### Synthesis of sodium salt of N-decyl-3-sulfoxybutyramide

### (1) Synthesis of N-decyl-3-hydroxybutyramide

535.7 g (3.41 mol) of n-decylamine was fed into a 2-ℓ round-bottomed flask equipped with a stirrer, a dropping funnel and a thermometer, and heated to 80°C while stirring under a stream of nitrogen gas. Then, from the dropping funnel, 293.2 g (3.41 mol) of γ-butyrolactone was introduced over a period of about 1 hr. During this period, the temperature of the reaction mixture elevated to about 100°C due to exothermic reaction. The obtained reaction mixture was further stirred at 100°C for 5 hrs. The obtained crude product having a temperature of its melting point or above was dropwise added to 3 ℓ of hexane to crystallize. Thus, 767.9 g of N-decyl-3-hydroxybutyramide was obtained (yield: 89.2%). The hydroxyl value thereof was 228.6 (calculated value: 230.5). The infrared absorption spectrum data and ¹H-NMR spectrum data thereof were as follows:

### Infrared absorption spectrum (KBr tablet method)

3304 (O-H stretching), 3104 (N-H stretching), 2924, 2856 (C-H stretching), 1642 (C=0 stretching) cm⁻¹

### ¹H-NMR spectrum (δ, ppm) in CDCl₃

a: 0.72 ppm (t, 3H)
b: 1.12 ppm (m, 14H)
c: 1.33 ppm (m, 2H)
d: 3.05 ppm (q, 2H)
e: 6.10 ppm (b, 1H)
f: 2.18 ppm (t, 2H)
g: 1.70 ppm (m, 2H)
h: 3.50 ppm (t, 2H)
i: 3.64 ppm (b, 1H)

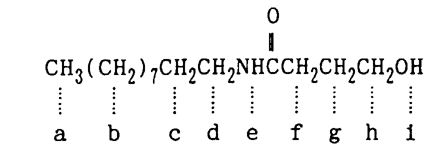

### (2) Synthesis of sodium salt of N-decyl-3-sulfoxybutyramide

750.9 g (2.97 mol) of the N-decyl-3-hydroxybutyramide synthesized in the above step (1) was put in a 5-ℓ round-bottomed flask equipped with a stirrer, a dropping funnel, a condenser tube and a thermometer. Next, 3 ℓ of chloroform was put in the flask to thereby dissolve the N-decyl-3-hydroxybutyramide in the chloroform. While stirring the obtained chloroform solution at room temperature under a stream of nitrogen gas, 363.5 g (3.12 mol) of chlorosulfonic acid was dropwise added thereto over a period of about 4 hrs. During the period, the flask was cooled in a water bath to prevent the reaction temperature from exceeding 40°C. Thereafter, the obtained mixture was further stirred at 20°C for 2 hrs. The obtained reaction mixture was poured into 3 ℓ of an ice water/n-butanol mixture containing 1000 g of ice water. The obtained mixture was stirred for about 10 min, and then transferred to a separatory funnel. An organic phase was separated. This organic phase was neutralized with a 20% aqueous sodium hydroxide solution to adjust the pH thereof to 7. Chloroform and butanol were distilled off from the organic phase under reduced pressure. The remaining solid was washed with 3 ℓ of ethanol to obtain 843.4 g of sodium salt of N-decyl-3-sulfoxybutyramide (yield: 82.2%). The infrared absorption spectrum data, ¹H-NMR spectrum data and mass spectrum data of the obtained compound were as follows:

### Infrared absorption spectrum (KBr tablet method)

3340 (N-H stretching), 2926, 2854 (C-H stretching), 1638 (C=O stretching), 1206, 1071 (S=0 stretching) cm⁻¹

### ¹H-NMR spectrum (δ, ppm) in D₂O

a: 0.88 ppm (t, 3H)
b: 1.30 ppm (m, 14H)
c: 1.50 ppm (m, 2H)
d: 3.20 ppm (t, 2H)
e: 2.37 ppm (t, 2H)
f: 1.96 ppm (m, 2H)
g: 4.10 ppm (t, 2H)

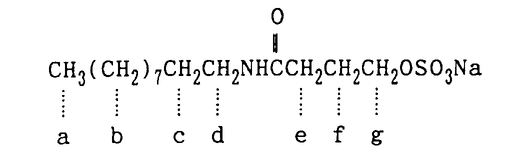

### Mass spectrum (Fab ionization method)

- 368:: (M+Na)⁺
- 266:: (M+H-SO₃)⁺
- 226:: (M-OSO₃Na)⁺

### Example 7

### Synthesis of sodium salt of N-tetradecyl-3-sulfoxybutyramide

### (1) Synthesis of N-tetradecyl-3-hydroxybutyramide

508.19 g (2.3813 mol) of tetradecylamine was fed into a 2-ℓ four-necked flask equipped with a stirrer, a dropping funnel and a thermometer, and heated to 90°C. Then, 208.4 g (2.421 mol) of γ-butyrolactone was dropwise added to the tetradecylamine over a period of about 0.5 hr. During this period, the temperature of the reaction system was maintained at 90 to 100°C. The obtained reaction mixture was further stirred at 100°C for 4 hrs. The obtained crude product having a temperature of its melting point or above was dropwise added to 2.5 ℓ of hexane to crystallize. Thus, 668.98 g of N-tetradecyl-3-hydroxybutyramide was obtained (yield: 94%). The hydroxyl value thereof was 190.7 (calculated value: 187.33). The infrared absorption spectrum data thereof were as follows:

### Infrared absorption spectrum (KBr tablet method)

3300 (O-H stretching), 3100 (N-H stretching), 2920, 2855 (C-H stretching), 1642 (C=O stretching) cm⁻¹

### (2) Synthesis of sodium salt of N-tetradecyl-3-sulfoxybutyramide

401.54 g (1.3407 mol) of the N-tetradecyl-3-hydroxybutyramide obtained in the above step (1) was put in a 5-ℓ round-bottomed flask equipped with a stirrer, a dropping funnel, a condenser tube and a thermometer. Next, 1.8 ℓ of chloroform was put in the flask to thereby dissolve the N-tetradecyl-3-hydroxybutyramide in the chloroform. While stirring the obtained chloroform solution at room temperature under a stream of nitrogen gas, 172.5 g (1.480 mol) of chlorosulfonic acid was dropwise added thereto over a period of about 1 hr. During the period, the temperature of the reaction system was maintained at 10 to 20°C. Thereafter, the obtained mixture was further stirred at room temperature for 3 hrs. The obtained reaction mixture was poured into a mixture of 1000 g of ice water and 1 ℓ of n-butanol. The obtained mixture was stirred for about 10 min, and transferred to a separatory funnel. An organic phase was separated. This organic phase was neutralized with a 30% aqueous NaOH solution to adjust the pH thereof to 7. Chloroform and butanol were distilled off from the organic phase under reduced pressure. The remaining solid was washed with 4 ℓ of acetone to obtain 511.43 g of sodium salt of N-tetradecyl-3-sulfoxybutyramide (yield: 95%). The infrared absorption spectrum data, ¹H-NMR spectrum data and mass spectrum data thereof were as follows:

### Infrared absorption spectrum (KBr tablet method)

3334 (N-H stretching), 2925, 2854 (C-H stretching), 1635 (C=0 stretching), 1206, 1070 (S=0 stretching) cm⁻¹

### ¹H-NMR spectrum (δ, ppm) in D₂O

a: 0.89 ppm (t, 3H)
b: 1.30 ppm (b, 22H)
c: 1.52 ppm (m, 2H)
d: 3.18 ppm (t, 2H)
e: 2.38 ppm (t, 2H)
f: 1.98 ppm (m, 2H)
g: 4.10 ppm (t, 2H)

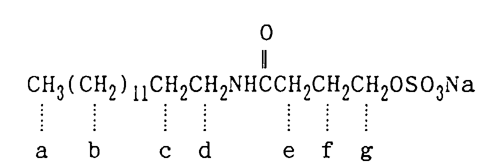

### Mass spectrum (Fab ionization method)

- 424:: (M+Na)⁺
- 322:: (M+H-SO₃)⁺
- 282:: (M-OSO₃Na)⁺

### Example 8

### Synthesis of ammonium salt of N-cocoyl-3-sulfoxybutyramide

### (1) Synthesis of N-cocoyl-3-hydroxybutyramide

777.36 g (3.9653 mol) of cocoyl alkylamine (Farmin CS produced by Kao Corp., having an average molecular weight of 196.04) was fed into a 2-ℓ four-necked flask equipped with a stirrer, a dropping funnel and a thermometer, and heated to 90°C. Then, 341.39 g (3.9655 mol) of γ-butyrolactone was dropwise added to the cocoyl alkylamine over a period of about 1 hr. During this period, the temperature of the reaction system was maintained at 90 to 100°C. The obtained reaction mixture was further stirred at 100°C for 3 hrs. The obtained crude product having a temperature of its melting point or above was dropwise added to 4 ℓ of hexane to crystallize. Thus, 1078.4 g of N-cocoyl-3-hydroxybutyramide was obtained (yield: 96%). The hydroxyl value thereof was 202.9 (calculated value: 198.86).

### (2) Synthesis of ammonium salt of N-cocoyl-3-sulfoxybutyramide

463.38 g (1.643 mol) of the N-cocoyl-3-hydroxybutyramide obtained in the above step (1) was put in a 5-ℓ round-bottomed flask equipped with a stirrer, a dropping funnel, a condenser tube and a thermometer. Next, 2 ℓ of chloroform was put in the flask to thereby dissolve the N-cocoyl-3-hydroxybutyramide in the chloroform. While stirring the obtained chloroform solution at room temperature under a stream of nitrogen gas, 202.0 g (1.734 mol) of chlorosulfonic acid was dropwise added thereto over a period of about 1.5 hrs. During the period, the temperature of the reaction system was maintained at 10 to 20°C. Thereafter, the obtained mixture was further stirred at room temperature for 2 hrs. The obtained reaction mixture was poured into a mixture of 1000 g of ice water and 1 ℓ of n-butanol. The obtained mixture was stirred for about 10 min, and transferred to a separatory funnel. An organic phase was separated. This organic phase was neutralized with 25% aqueous ammonia to adjust the pH thereof to 7. Chloroform and butanol were distilled off from the organic phase under reduced pressure. The remaining solid was washed with 4 ℓ of acetone to obtain 591.91 g of ammonium salt of N-cocoyl-3-sulfoxybutyramide (yield: 95%). The infrared absorption spectrum data thereof were as follows:

### Infrared absorption spectrum (KBr tablet method)

3260 (N-H stretching), 2926, 2855 (C-H stretching), 1634 (C=0 stretching), 1210, 1085 (S=0 stretching) cm⁻¹

### Example 9

### Synthesis of sodium salt of N-dodecyl-2-methyl-4-sulfoxypentanamide

216.6 g (1.17 mol) of dodecylamine was fed into a 2-ℓ round-bottomed flask equipped with a stirrer, a dropping funnel, a condenser tube and a thermometer, and heated to 90°C under a stream of nitrogen gas. Then, from the dropping funnel, 133.4 g (1.17 mol) of 2-methyl-δ-valerolactone was introduced over a period of about 1 hr while stirring the dodecylamine. During this period, the temperature of the reaction mixture elevated to about 100°C due to exothermic reaction. The obtained reaction mixture was further stirred at 100°C for 5 hrs. Without isolating an alkanolamide, a sulfation reaction was carried out. More specifically, the reaction mixture was cooled to room temperature, and then 1000 g of methylene chloride was added to dissolve the same. At room temperature, 143.0 g (1.23 mol) of chlorosulfonic acid was dropwise added to the obtained methylene chloride solution over a period of about 1 hr. After the completion of the dropwise addition, the obtained mixture was stirred at 30°C for about 2 hr. Then, the reaction mixture was poured into a mixture of 500 g of ice water and 500 ml of n-butanol. The obtained mixture was transferred to a separatory funnel, the funnel was vigorously shaken and then an organic phase was separated. This organic phase was neutralized with a 30% aqueous NaOH solution to adjust the pH thereof to 7.2. The solvent and water were distilled off from the organic phase under reduced pressure, and then the residue was washed with ethanol. Thus, 417.4 g of sodium salt of N-dodecyl-2-methyl-4-sulfoxypentanamide was obtained (yield: 88.7%). The infrared absorption spectrum data and ¹H-NMR spectrum data thereof were as follows:

### Infrared absorption spectrum (KBr tablet method)

3262 (N-H stretching), 2926, 2854 (C-H stretching), 1635 (C=0 stretching), 1209, 1083 (S=0 stretching) cm⁻¹

### ¹H-NMR spectrum (δ, ppm) in D₂O

- a, i:: 0.60 ppm (m, 6H)
- b:: 0.93 ppm (m, 18H)
- c:: 1.33 ppm (m, 2H)
- d, f:: 2.90 ppm (m, 3H)
- e:: 2.03 ppm (d, 2H)
- g:: 1.74 ppm (m, 2H)
- h:: 3.78 ppm (t, 2H)

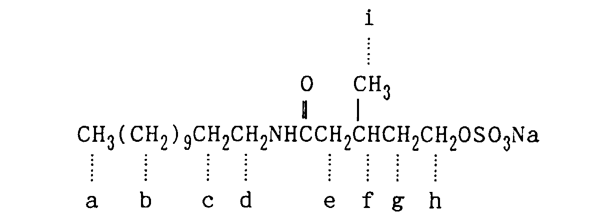

### Example 10

### Synthesis of sodium salt of N-octyl-4-sulfoxypentanamide

### (1) Synthesis of N-octyl-4-hydroxypentanamide

34.14 g (0.8641 mol) of octylamine was fed into a 100-ml four-necked flask equipped with a stirrer, a dropping funnel and a thermometer, and heated to 90°C. Then, 26.46 g (0.2643 mol) of δ-valerolactone was dropwise added to the octylamine over a period of about 10 min. During this period, the temperature of the reaction system was maintained at 90 to 100°C. The obtained reaction mixture was further stirred at 100°C for 4 hrs. The obtained crude product having a temperature of its melting point or above was dropwise added to 400 ml of hexane to crystallize. Thus, 46.58 g of N-octyl-4-hydroxypentanamide was obtained (yield: 77%). The infrared absorption spectrum data thereof were as follows:

### Infrared absorption spectrum (KBr tablet method)

3320 (O-H stretching), 2922, 2856 (C-H stretching), 1636 (C=0 stretching) cm⁻¹

### (2) Synthesis of sodium salt of N-octyl-4-sulfoxypentanamide

40.29 g (0.1757 mol) of the N-octyl-4-hydroxypentanamide obtained in the above step (1) was put in a 300-ml round-bottomed flask equipped with a stirrer, a dropping funnel, a condenser tube and a thermometer. Next, 160 ml of chloroform was put in the flask to thereby dissolve the N-octyl-4-hydroxypentanamide in the chloroform. While stirring the obtained chloroform solution at room temperature under a stream of nitrogen gas, 22.5 g (0.193 mol) of chlorosulfonic acid was dropwise added thereto over a period of about 20 min. During the period, the temperature of the reaction system was maintained at 10 to 20°C. Thereafter, the obtained mixture was further stirred at room temperature for 1 hr. The obtained reaction mixture was poured into a mixture of 150 g of ice water and 70 ml of n-butanol. The obtained mixture was stirred for about 10 min, and transferred to a separatory funnel. An organic phase was separated. Next, this organic phase was neutralized with a 30% aqueous NaOH solution to adjust the pH thereof to 7. Chloroform and butanol were distilled off from the organic phase under reduced pressure. The remaining solid was washed with 200 ml of acetone to obtain 44.84 g of sodium salt of N-octyl-4-sulfoxypentanamide (yield: 77%). The infrared absorption spectrum data, ¹H-NMR spectrum data and mass spectrum data thereof were as follows:

### Infrared absorption spectrum (KBr tablet method)

3345 (N-H stretching), 2926, 2855 (C-H stretching), 1640 (C=0 stretching), 1200, 1077 (S=0 stretching) cm⁻¹

### ¹H-NMR spectrum (δ, ppm) in D₂O

a: 0.95 ppm (t, 3H)
b: 1.28 ppm (b, 10H)
c: 1.53 ppm (m, 2H)
d: 3.18 ppm (t, 2H)
e: 2.30 ppm (t, 2H)
f: 1.75 ppm (m, 4H)
g: 4.10 ppm (t, 2H)

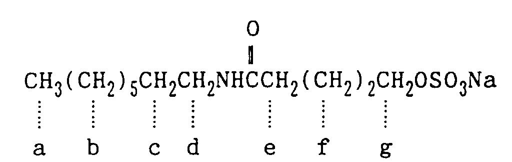

### Mass spectrum (Fab ionization method)

- 354:: (M+Na)⁺
- 252:: (M+H-SO₃)⁺
- 212:: (M-OSO₃Na)⁺

### Example 11

### Synthesis of sodium salt of N-dodecyl-4-sulfoxypentanamide

### (1) Synthesis of N-dodecyl-4-hydroxypentanamide

49.77 g (0.2685 mol) of dodecylamine was fed into a 100-ml four-necked flask equipped with a stirrer, a dropping funnel and a thermometer, and heated to 90°C. Then, 26.89 g (0.2686 mol) of δ-valerolactone was dropwise added to the dodecylamine over a period of about 15 min. During this period, the temperature of the reaction system was maintained at 90 to 100°C. The obtained reaction mixture was further stirred at 100°C for 3 hrs. The obtained crude product having a temperature of its melting point or above was dropwise added to 400 ml of hexane to crystallize. Thus, 57.95 g of N-dodecyl-4-hydroxypentanamide was obtained (yield: 76%). The hydroxyl value thereof was 183.1 (calculated value: 196.54). The infrared absorption spectrum data thereof were as follows:

### Infrared absorption spectrum (KBr tablet method)

3332 (O-H stretching), 2924, 2856 (C-H stretching), 1636 (C=0 stretching) cm⁻¹

### (2) Synthesis of sodium salt of N-dodecyl-4-sulfoxypentanamide

50.21 g (0.1759 mol) of the N-dodecyl-4-hydroxypentanamide obtained in the above step (1) was put in a 500-ml round-bottomed flask equipped with a stirrer, a dropping funnel, a condenser tube and a thermometer. Next, 200 ml of chloroform was put in the flask to thereby dissolve the N-dodecyl-4-hydroxypentanamide in the chloroform. While stirring the obtained chloroform solution at room temperature under a stream of nitrogen gas, 21.6 g (0.185 mol) of chlorosulfonic acid was dropwise added thereto over a period of about 20 min. During the period, the temperature of the reaction system was maintained at 10 to 20°C. Thereafter, the obtained mixture was further stirred at room temperature for 2 hrs. The obtained reaction mixture was poured into a mixture of 100 g of ice water and 100 ml of n-butanol. The obtained mixture was stirred for about 10 min, and transferred to a separatory funnel. An organic phase was separated. This organic phase was neutralized with a 30% aqueous NaOH solution to adjust the pH thereof to 7. Chloroform and butanol were distilled off from the organic phase under reduced pressure. The remaining solid was washed with 500 ml of acetone to obtain 51.57 g of sodium salt of N-dodecyl-4-sulfoxypentanamide (yield: 76%). The infrared absorption spectrum data, ¹H-NMR spectrum data and mass spectrum data thereof were as follows:

### Infrared absorption spectrum (KBr tablet method)

3346 (N-H stretching), 2926, 2854 (C-H stretching), 1641 (C=0 stretching), 1197, 1077 (S=0 stretching) cm⁻¹

### ¹H-NMR spectrum (δ, ppm) in D₂O

a: 0.65 ppm (t, 3H)
b: 1.05 ppm (b, 18H)
c: 1.28 ppm (m, 2H)
d: 2.89 ppm (t, 2H)
e: 2.10 ppm (t, 2H)
f: 1.49 ppm (m, 4H)
g: 3.75 ppm (t, 2H)

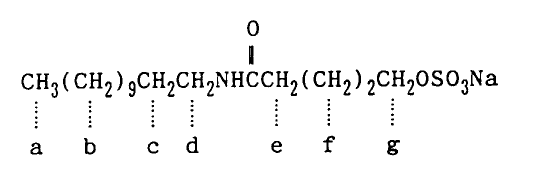

### Mass spectrum (Fab ionization method)

- 410:: (M+(Na)⁺
- 308:: (M+H-SO₃)⁺
- 268:: (M-OSO₃Na)⁺

### Example 12

### Synthesis of sodium salt of N-decyl-5-sulfoxyhexanamide

### (1) Synthesis of N-decyl-5-hydroxyhexanamide

362.56 g (2.3049 mol) of decylamine was fed into a 1-ℓ four-necked flask equipped with a stirrer, a dropping funnel and a thermometer, and heated to 90°C. Then, 263.08 g (2.3049 mol) of ε-caprolactone was dropwise added to the decylamine over a period of about 30 min. During this period, the temperature of the reaction system was maintained at 90 to 100°C. The obtained reaction mixture was further stirred at 100°C for 5 hrs. The obtained crude product having a temperature of itsmelting point or above was dropwise added to 2 ℓ of hexane to crystallize. Thus, 528.69 g of N-decyl-5-hydroxyhexanamide was obtained (yield: 85%). The hydroxyl value thereof was 181.6 (calculated value: 206.70). The infrared absorption spectrum data thereof were as follows:

### Infrared absorption spectrum (KBr tablet method)

3321 (O-H stretching), 2926, 2855 (C-H stretching), 1635 (C=0 stretching) cm⁻¹

### (2) Synthesis of sodium salt of N-decyl-5-sulfoxyhexanamide

467.58 g (1.7226 mol) of the N-decyl-5-hydroxyhexanamide obtained in the above step (1) was put in a 5-ℓ round-bottomed flask equipped with a stirrer, a dropping funnel, a condenser tube and a thermometer. Next, 2 ℓ of chloroform was put in the flask to thereby dissolve the N-decyl-5-hydroxyhexanamide in the chloroform. While stirring the obtained chloroform solution at room temperature under a stream of nitrogen gas, 210.9 g (1.810 mol) of chlorosulfonic acid was dropwise added thereto over a period of about 1.5 hrs. During the period, the temperature of the reaction system was maintained at 10 to 20°C. Thereafter, the obtained mixture was further stirred at room temperature for 2 hrs. The obtained reaction mixture was poured into a mixture of 1000 g of ice water and 500 ml of n-butanol. The obtained mixture was stirred for about 10 min, and transferred to a separatory funnel. An organic phase was separated. This organic phase was neutralized with a 30% aqueous NaOH solution to adjust the pH thereof to 7. Chloroform and butanol were distilled off from the organic phase under reduced pressure. The remaining solid was washed with 4 ℓ of acetone to obtain 521.16 g of sodium salt of N-decyl-5-sulfoxyhexanamide (yield: 81%). The infrared absorption spectrum data, ¹H-NMR spectrum data and mass spectrum data of the obtained compound were as follows:

### Infrared absorption spectrum (KBr tablet method)

3316 (N-H stretching), 2926, 2854 (C-H stretching), 1635 (C=0 stretching), 1206, 1071 (S=0 stretching) cm⁻¹

### ¹H-NMR spectrum (δ, ppm) in D₂O

a: 0.92 ppm (t, 3H)
b: 1.38 ppm (b, 14H)
c: 1.50 ppm (m, 2H)
d: 3.19 ppm (t, 2H)
e: 2.29 ppm (t, 2H)
f: 1.71 ppm (m, 6H)
g: 4.07 ppm (t, 2H)

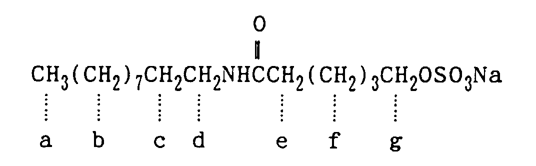

### Mass spectrum (Fab ionization method)

- 396:: (M+Na)⁺
- 294:: (M+H-SO₃)⁺
- 254:: (M-OSO₃Na)⁺

### Example 13

### Synthesis of sodium salt of N-dodecyl-2-sulfoxypropionamide

### (1) Synthesis of N-dodecyl-2-hydroxypropionamide

25.34 g (0.1367 mol) of dodecylamine and 30 g of dioxane were fed into a 200-ml four-necked flask equipped with a stirrer, a dropping funnel and a thermometer to thereby prepare a transparent solution. Then, 9.85 g (0.1367 mol) of β-propiolactone was dropwise added to the transparent solution over a period of about 15 min. During this period, the temperature of the reaction system was maintained at 25 to 30°C. The obtained reaction mixture was further stirred at 30°C for 5 hrs. The obtained crude product having a temperature of its melting pointor above was dropwise added to 100 ml of hexane to crystallize. Thus, 28.93 g of N-dodecyl-2-hydroxypropionamide was obtained (yield: 82%). The infrared absorption spectrum data and ¹H-NMR spectrum data of the obtained compound were as follows:

### Infrared absorption spectrum (KBr tablet method)

3298 (O-H stretching), 2926, 2854 (C-H stretching), 1644 (C=0 stretching) cm⁻¹

### ¹H-NMR spectrum (δ, ppm) in CDCl₃

a: 0.82 ppm (t, 3H)
b: 1.35 ppm (b, 18H)
c: 1.47 ppm (m, 2H)
d: 3.11 ppm (q, 2H)
e: 6.20 ppm (b, 2H)
f: 2.30 ppm (t, 2H)
g: 3.79 ppm (t, 2H)
h: 5.00 ppm (b, 1H)

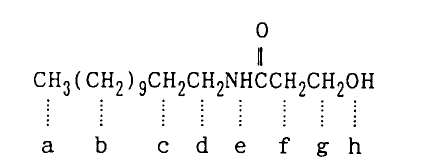

### (2) Synthesis of sodium salt of N-dodecyl-2-sulfoxypropionamide

15.34 g (0.05959 mol) of the N-dodecyl-2-hydroxypropionamide obtained in the above step (1) was put in a 100-ml round-bottomed flask equipped with a stirrer, a dropping funnel, a condenser tube and a thermometer. Next, 100 ml of chloroform was put in the flask to thereby dissolve the N-dodecyl-2-hydroxypropionamide in the chloroform. While stirring the obtained chloroform solution at room temperature under a stream of nitrogen gas, 7.67 g (0.0658 mol) of chlorosulfonic acid was dropwise added thereto over a period of about 10 min. During the period, the temperature of the reaction system was maintained at 10 to 20°C. Thereafter, the obtained mixture was further stirred at room temperature for 1 hr. The obtained reaction mixture was poured into a mixture of 50 g of ice water and 50 ml of n-butanol. The obtained mixture was stirred for about 10 min, and transferred to a separatory funnel. An organic phase was separated. This organic phase was neutralized with a 20% aqueous NaOH solution to adjust the pH thereof to 7.
Chloroform and butanol were distilled off from the organic phase under reduced pressure. The remaining solid was washed with 100 ml of acetone to obtain 19.32 g of sodium salt of N-dodecyl-2-sulfoxypropionamide (yield: 90%). The infrared absorption spectrum data, ¹H-NMR spectrum data and mass spectrum data of the obtained compound were as follows:

### Infrared absorption spectrum (KBr tablet method)

3272 (N-H stretching), 2928, 2856 (C-H stretching), 1636 (C=O stretching), 1208, 1084 (S=0 stretching) cm⁻¹

### ¹H-NMR spectrum (δ, ppm) in D₂O

a: 0.89 ppm (t, 3H)
b: 1.35 ppm (b, 18H)
c: 1.58 ppm (m, 2H)
d: 3.20 ppm (t, 2H)
e: 2.65 ppm (t, 2H)
f: 4.27 ppm (t, 2H)

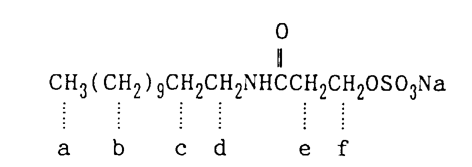

### Mass spectrum (Fab ionisation method)

- 382:: (M+Na)⁺
- 280:: (M+H-SO₃)⁺
- 240:: (M-OSO₃Na)⁺

### Example 14

### Synthesis of potassium salt of N-dodecyl-2-methyl-4-sulfoxypentanamide

150.0 g (0.81 mol) of dodecylamine was fed into a 1-ℓ round-bottomed flask equipped with a stirrer, a dropping funnel, a condenser tube and a thermometer, and heated to 90°C under a stream of nitrogen gas. Then, from the dropping funnel, 92.4 g (0.81 mol) of 2-methyl-δ-valerolactone was introduced over a period of about 1 hr while stirring the dodecylamine. During this period, the temperature of the reaction mixture elevated to about 100°C due to exothermic reaction. The obtained reaction mixture was further stirred at 100°C for 5 hrs. Without isolating an alkanolamide, a sulfation reaction was carried out. More specifically, the reaction mixture was cooled to room temperature, and 500 g of methylene chloride was added to dissolve the same. At room temperature, 99.0 g (0.85 mol) of chlorosulfonic acid was dropwise added to the obtained methylene chloride solution over a period of about 1 hr. After the completion of the dropwise addition, the obtained mixture was stirred at 30°C for about 2 hrs. The obtained reaction mixture was poured into a mixture of 500 g of ice water and 500 ml of butanol. The obtained mixture was transferred to a separatory funnel and the funnel was vigorously shaken. An organic phase was separated. The organic phase was neutralized with a 30% aqueous KOH solution to adjust the pH thereof to 7.0. The solvent and water were distilled off from the organic phase under reduced pressure, and the residue was washed with ethanol. Thus, 308.6 g of potassium salt of N-dodecyl-2-methyl-4-sulfoxypentanamide was obtained (yield: 91.0%). The infrared absorption spectrum data and ¹H-NMR spectrum data of the obtained compound were as follows:

### Infrared absorption spectrum (KBr tablet method)

3262 (N-H stretching), 2926, 2854 (C-H stretching), 1635 (C=0 stretching), 1210, 1085 (S=0 stretching) cm⁻¹

### ¹H-NMR spectrum (δ, ppm) in D₂O

- a, i:: 0.60 ppm (m, 6H)
- b:: 0.93 ppm (m, 18H)
- c:: 1.33 ppm (m, 2H)
- d, f:: 2.90 ppm (m, 3H)
- e:: 2.03 ppm (d, 2H)
- g:: 1.74 ppm (m, 2H)
- h:: 3.78 ppm (t, 2H)

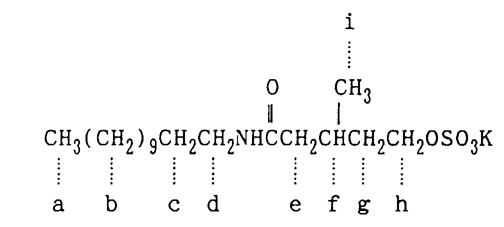

### Example 15

### Synthesis of sodium salt of N-decylsulfoxypolyoxyethylene(EO=5)butyramide

252.8 g (1.0 mol) of the N-decyl-3-hydroxybutyramide synthesized in step (1) of Example 6 and 2 g of sodium methylate were fed into a hermetically closed reactor vessel equipped with a stirrer, a dropping funnel and a thermometer. After purging the air in the reactor vessel with nitrogen gas, the mixture in the vessel was heated to 100°C. Then, 220.0 g (5.0 mol) of ethylene oxide was introduced to the vessel at that temperature. The obtained mixture was stirred at 100°C for 5 hrs. Next, the reaction mixture was cooled to room temperature, and 116.5 g (1.0 mol) of chlorosulfonic acid was added thereto over a period of 2 hrs. The obtained reaction mixture was further stirred at 30°C for 1 hr, and poured into 300 g of ice water. The resultant mixture was neutralized with a 30% aqueous sodium hydroxide solution to adjust the pH thereof to 7.0. The solvent and water were distilled off from the mixture under reduced pressure, thereby obtaining 550 g of sodium salt of N-decylsulfoxypolyoxyethylene(EO=5)butyramide having 5 mol (average value) of ethylene oxide added thereto (yield: 98.6%). The sulfate purity thereof was found to be 95.5% by measurement according to the Epton method. The infrared absorption spectrum data thereof were as follows:

### Infrared absorption spectrum (nujol method)

3341 (N-H stretching), 2925, 2855 (C-H stretching), 1638 (C=0 stretching), 1207, 1070 (S=0 stretching) cm⁻¹

### Testing Example 1

The foaming power of each of the salts of N-alkylcarbamylalkanol sulfate according to the present invention and comparative product listed in Table 1 was measured by the following method. The results are also shown in Table 1.

### <Method for testing foaming power>

To 4° DH hard water at 40°C, each of the surfactants together with lanolin was added to prepare an aqueous solution. The concentrations of the surfactant and the lanolin in the solution were 0.1% by weight and 0.3% by weight, respectively. The aqueous solution was stirred by the reversing stirring technique at 1450 rpm for 6 min. The volume of foam formed and remaining 10 sec and 120 sec after the termination of the stirring were measured.

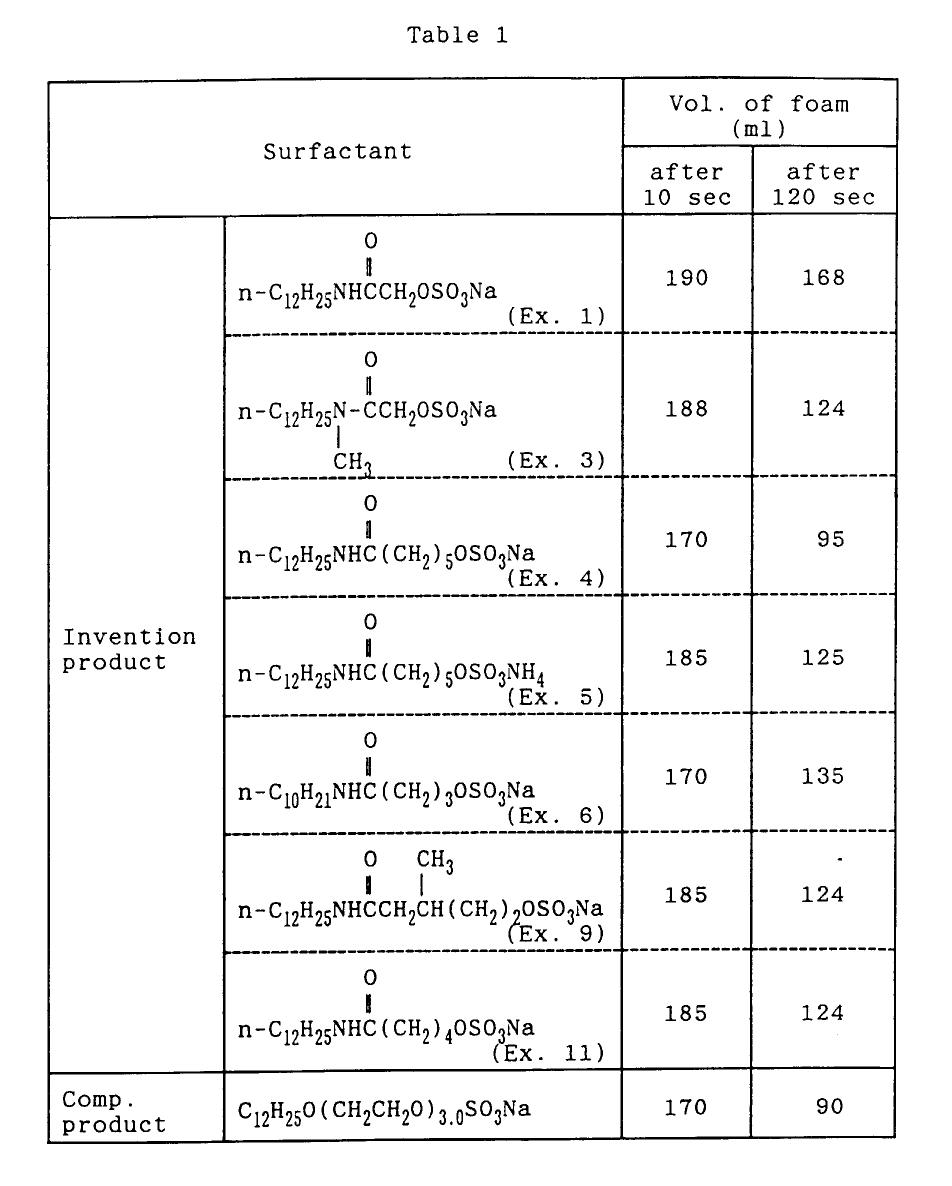

### Testing Example 2

With respect to each of the salts of N-alkylcarbamylalkanol sulfate according to the present invention and comparative products listed in Table 2, the irritancy to the skin was measured by the following quadruple cumulative irritation testing method. The results are also shown in Table 2.

### <Quadruple cumulative irritation testing method>

An aqueous solution containing 10% by weight of each of the surfactants was applied to the healthy skin of each of five guinea pigs four times. The reaction of the skin after the fourth application of the aqueous solution was evaluated according to the following criteria. Table 2 shows the average evaluation marks.

### <Evaluation criteria>

0: no reaction was observed;
1: slight erythema was observed;
2: clear erythema was observed;
3: clear erythema accompanied by edema was observed;
   and
4: clear erythema accompanied by necrosis or asphyxia was observed.

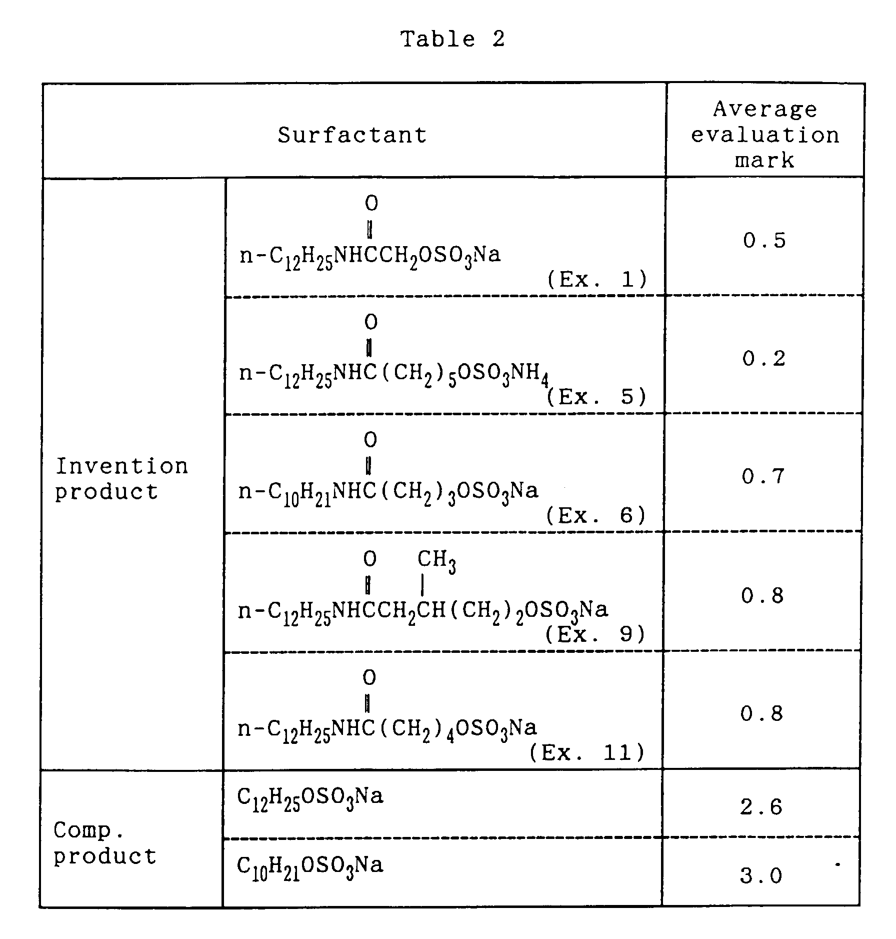

Hereinbelow, formulation examples of the detergent compositions of the present invention will be described.

### Formulation Example 1

A shampoo having the composition given below was prepared. The obtained shampoo not only was excellent in foaming power and detergency, but also ensured good feeling at the time of washing and rinsing of the hair.

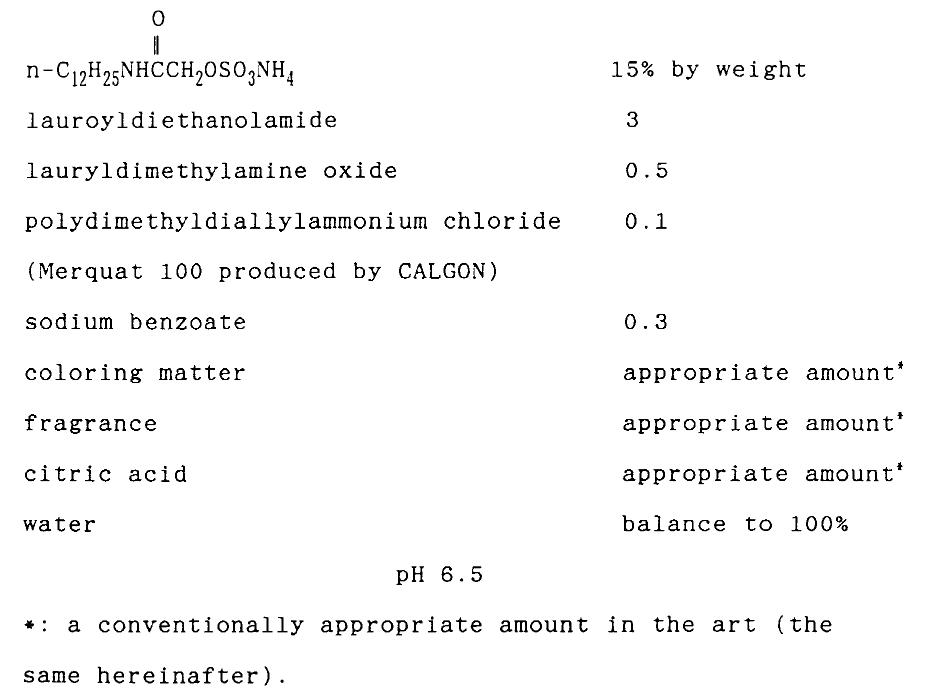

### Formulation Example 2

A shampoo having the composition given below was prepared. The obtained shampoo not only was excellent in foaming power and detergency, but also ensured good feeling at the time of washing and rinsing of the hair.

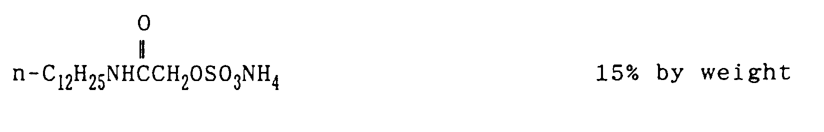

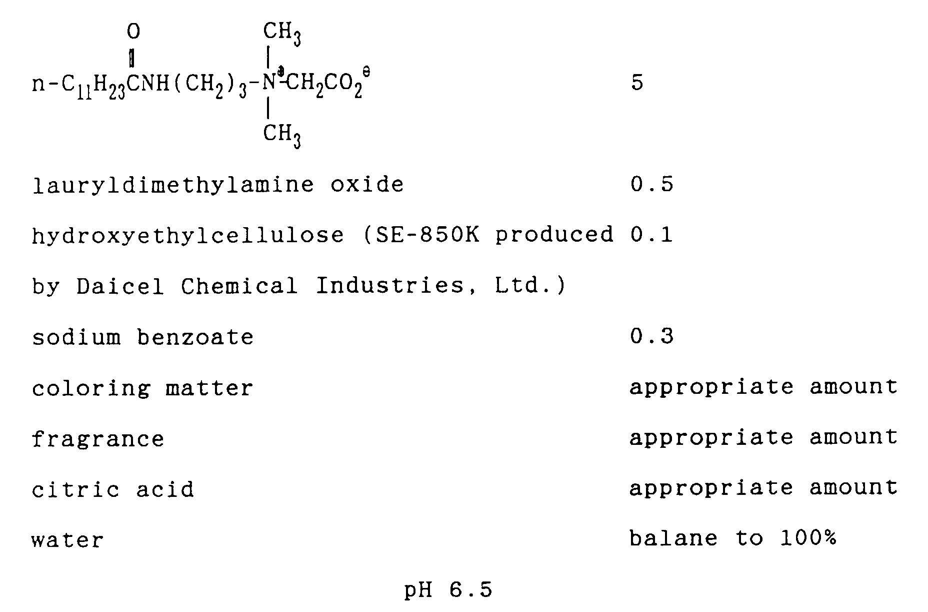

### Formulation Example 3

A shampoo having the composition given below was prepared. The obtained shampoo not only was excellent in foaming power and detergency, but also ensured good feeling without squeak at the time of washing and rinsing of the hair.

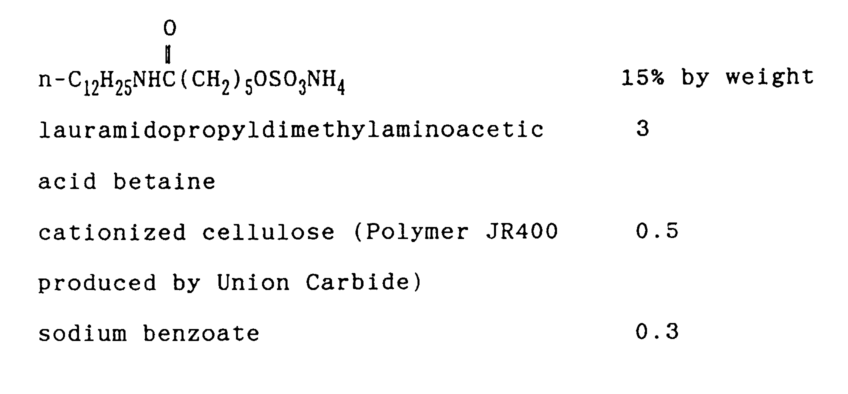

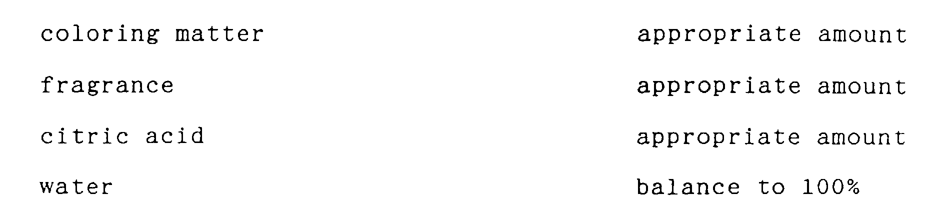

### Formulation Example 4

A shampoo having the composition given below was prepared. The obtained shampoo not only was excellent in foaming power and detergency, but also ensured good feeling without squeak at the time of washing and rinsing of the hair.

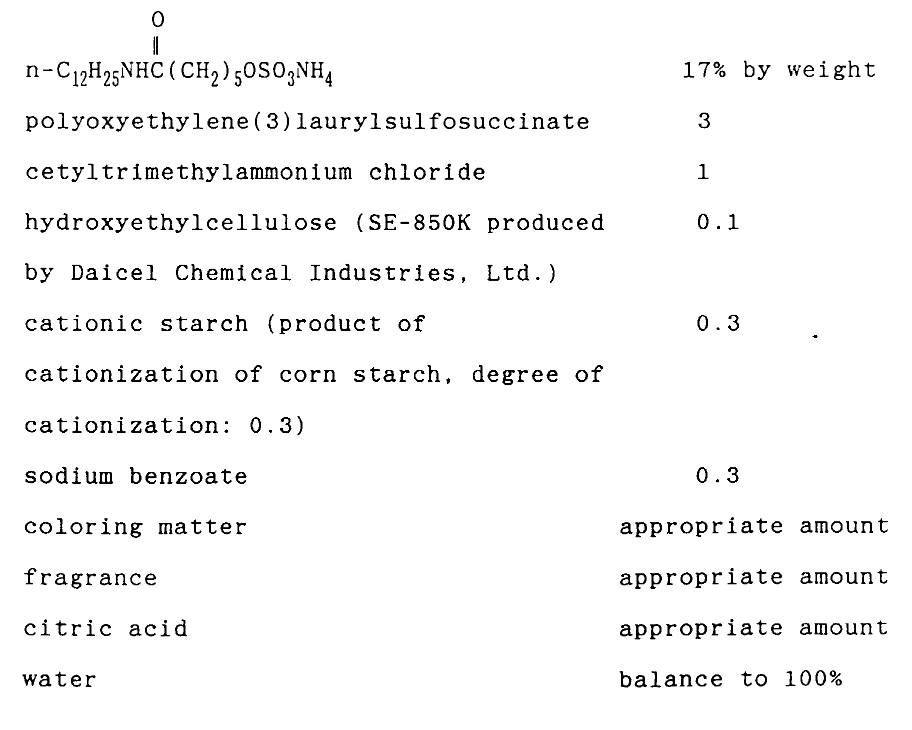

### Formulation Example 5

A body shampoo having the composition given below was prepared. The obtained body shampoo not only was excellent in foaming power and detergency, but also ensured moist and good feeling after washing.

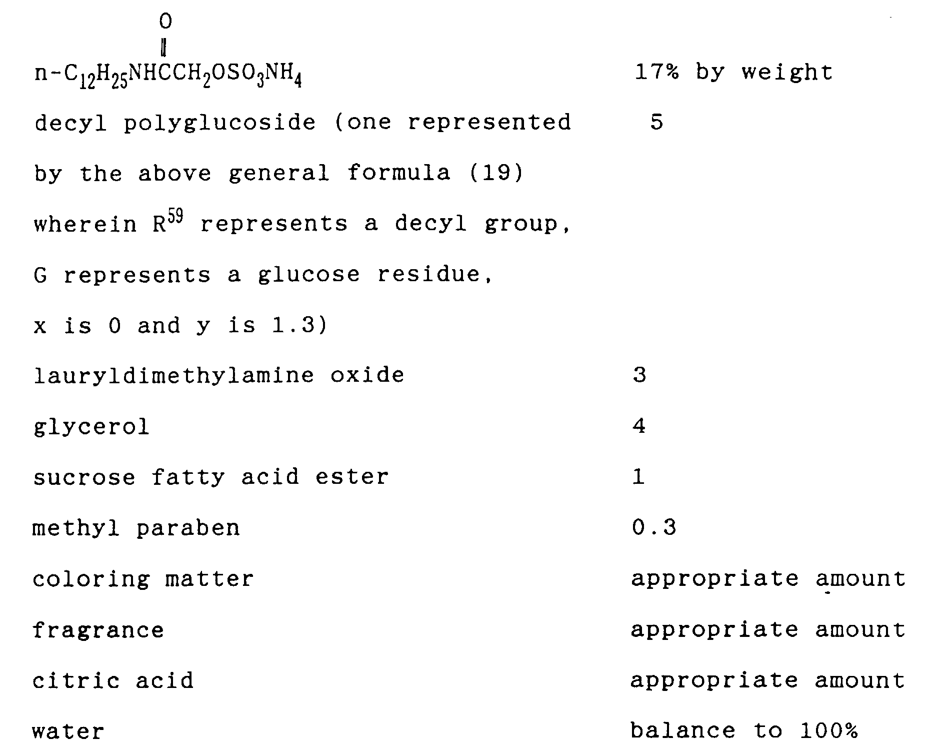

### Formulation Example 6

A shampoo having the composition given below was prepared. The obtained shampoo not only was excellent in foaming power and detergency, but also ensured good feeling at the time of washing and rinsing of the hair.

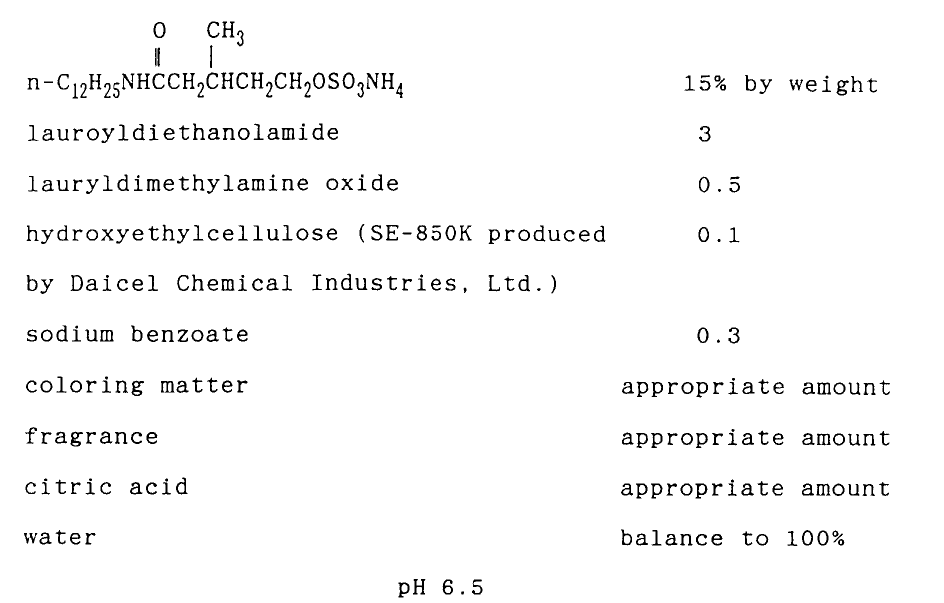

### Formulation Example 7

A shampoo having the composition given below was prepared. The obtained shampoo not only was excellent in foaming power and detergency, but also ensured good feeling at the time of washing and rinsing of the hair.

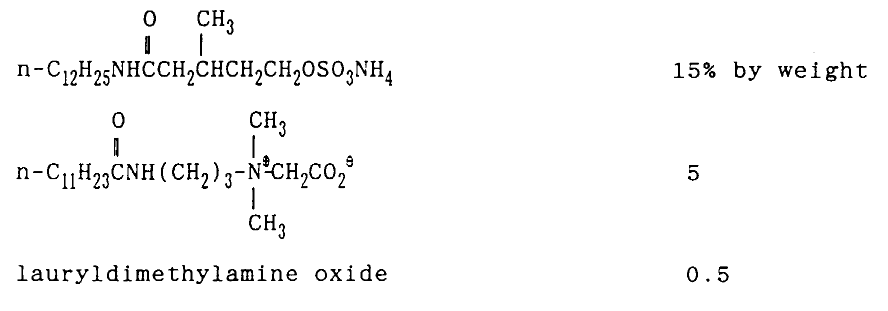

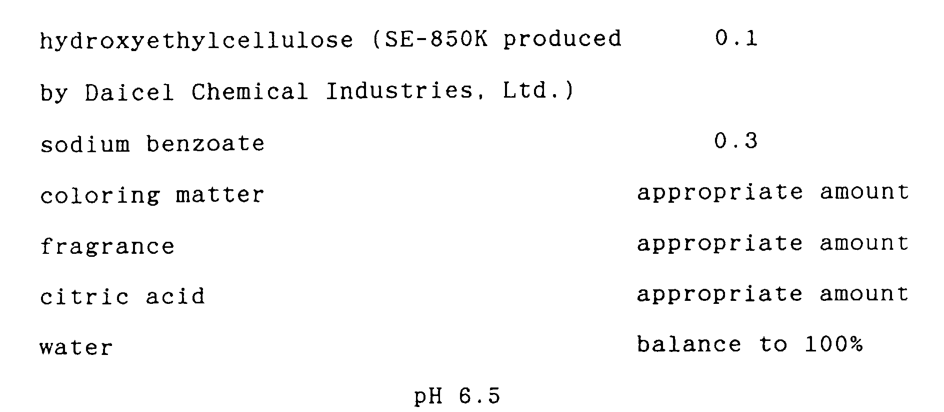

### Formulation Example 8

A shampoo having the composition given below was prepared. The obtained shampoo not only was excellent in foaming power and detergency, but also ensured good feeling without squeak at the time of washing and rinsing of the hair.

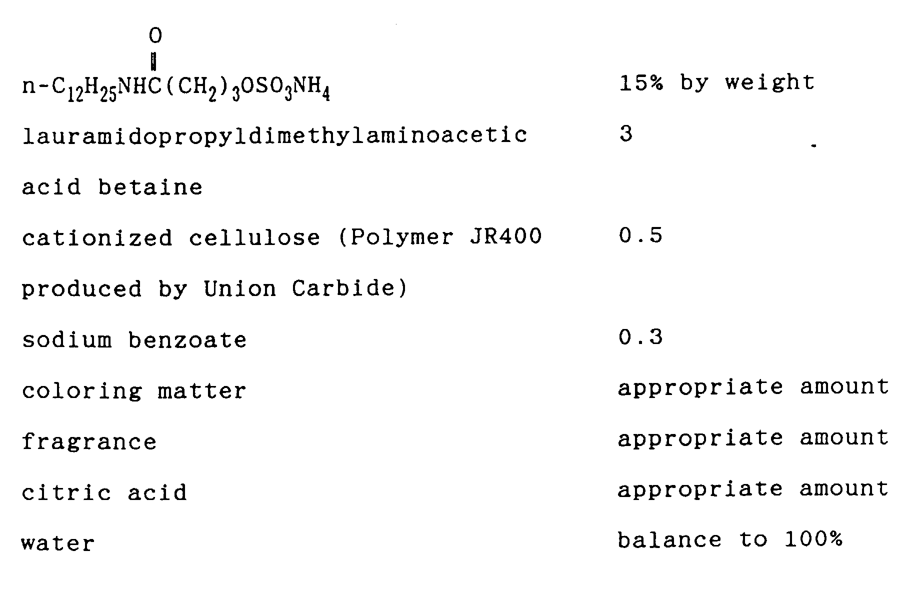

### Formulation Example 9

A shampoo having the composition given below was prepared. The obtained shampoo not only was excellent in foaming power and detergency, but also ensured good feeling without squeak at the time of washing and rinsing of the hair.

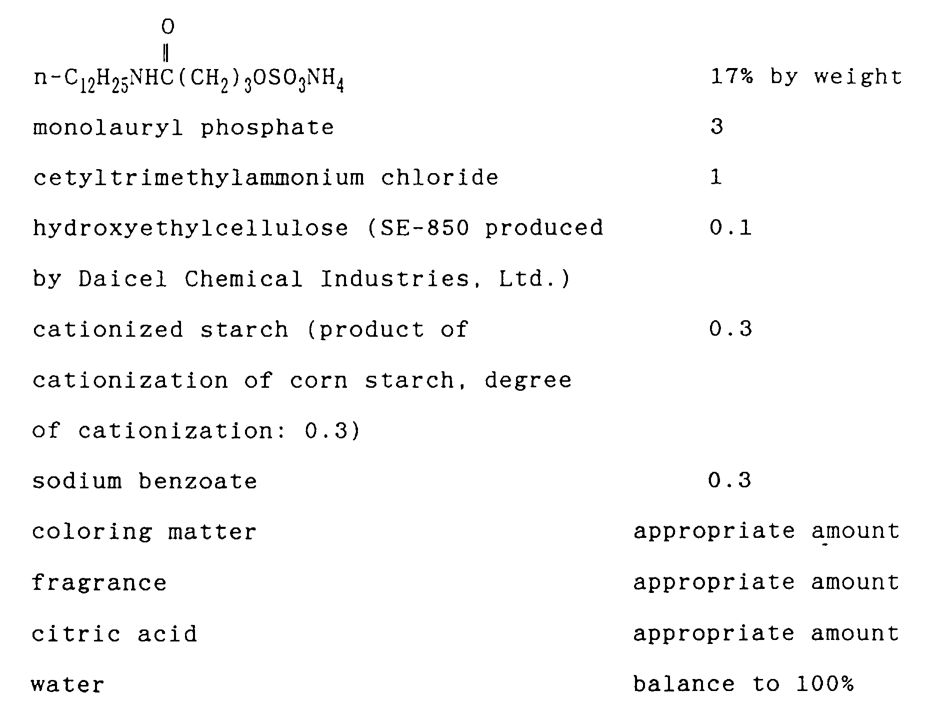

### Formulation Example 10

A body shampoo having the composition given below was prepared. The obtained body shampoo not only was excellent in foaming power and detergency, but also ensured moist and good feeling after washing.

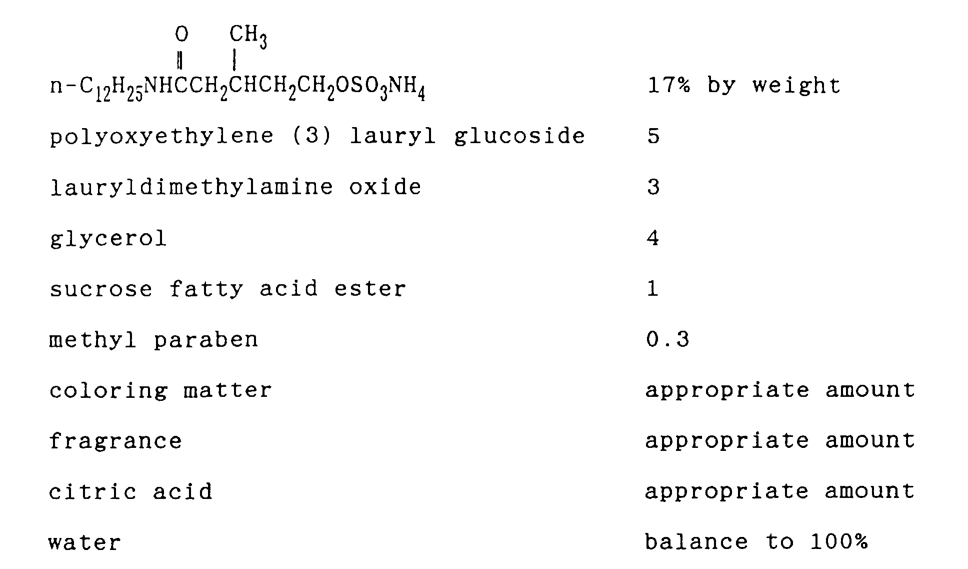

### Formulation Example 11

A shampoo having the composition given below was prepared. The obtained shampoo not only was excellent in foaming power and detergency, but also ensured good feeling without squeak at the time of washing and rinsing of the hair. Moreover, the shampoo exhibited excellent conditioning effect, and was especially excellent in slipperiness after drying.

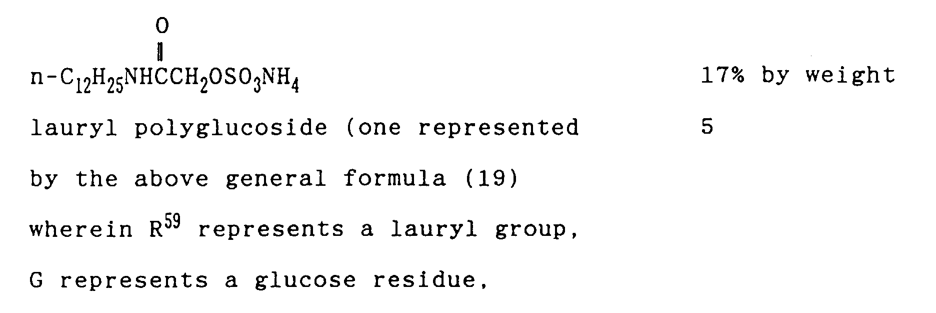

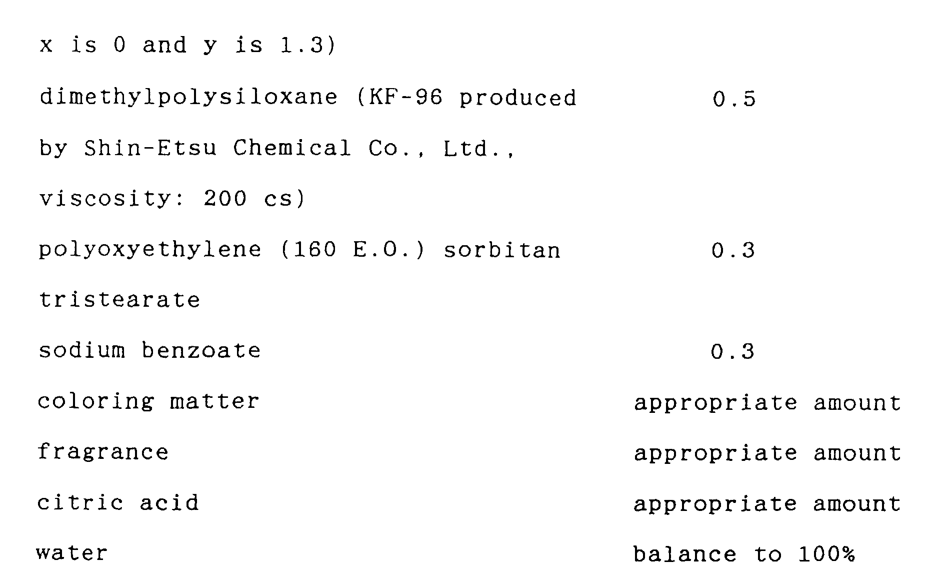

### Formulation Example 12

A shampoo having the composition given below was prepared. The obtained shampoo not only was excellent in foaming power and detergency, but also ensured good feeling without squeak at the time of washing and rinsing of the hair. Moreover, the shampoo exhibited excellent conditioning effect, and ensured excellent slipperiness and flexibility at the time of rinsing and after drying.

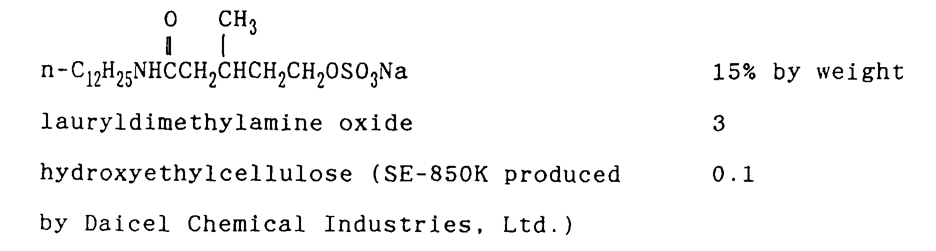

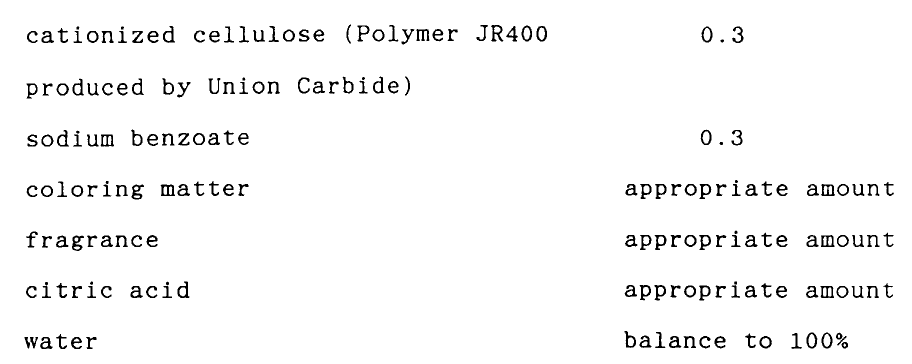

### Formulation Example 13

A shampoo having the composition given below was prepared. The obtained shampoo not only was excellent in foaming power and detergency, but also ensured good feeling without squeak at the time of washing and rinsing of the hair. Moreover, the shampoo exhibited excellent conditioning effect, and ensured excellent slipperiness and flexibility at the time of rinsing and after drying.

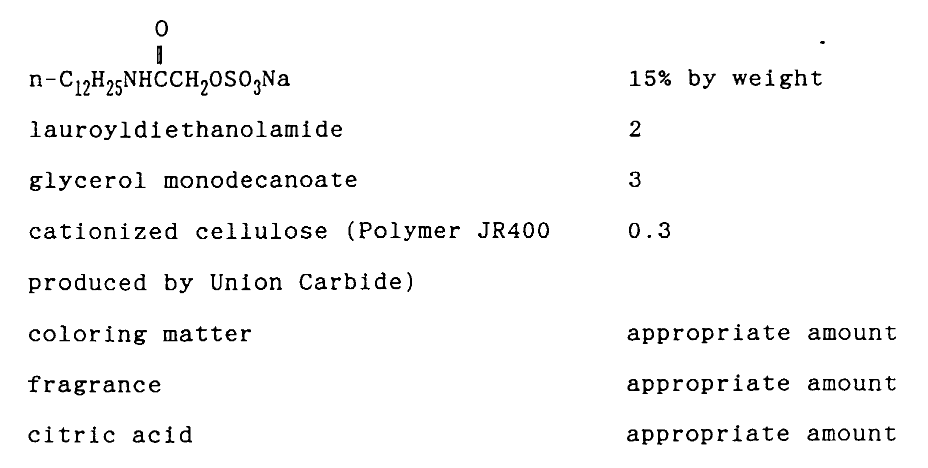

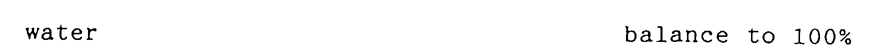

### Formulation Example 14

A shampoo having the composition given below was prepared. The obtained shampoo not only was excellent in foaming power and detergency, but also ensured good feeling without squeak at the time of washing and rinsing of the hair. Moreover, the shampoo exhibited excellent conditioning effect, and ensured excellent slipperiness and flexibility at the time of rinsing and after drying.

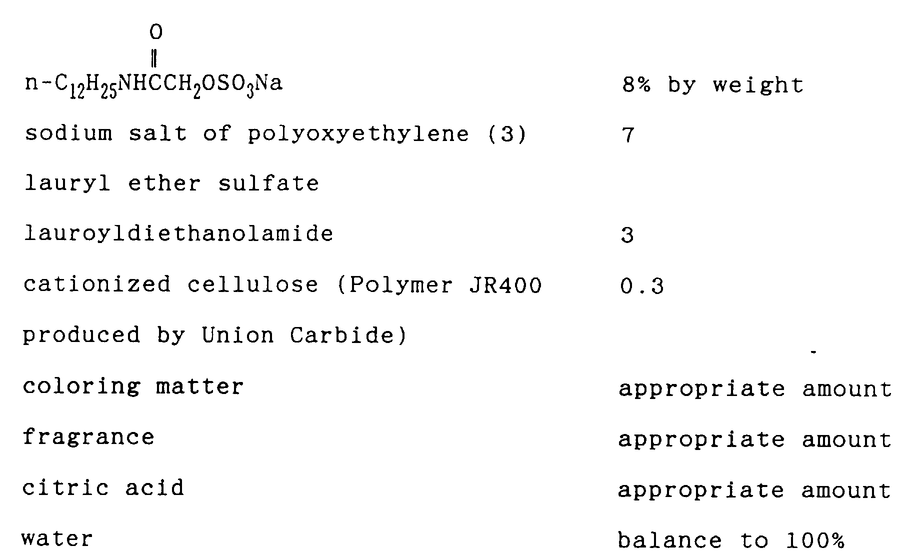

### Formulation Example 15

A shampoo having the composition given below was prepared. The obtained shampoo not only was excellent in foaming power and detergency, but also ensured good feeling without squeak at the time of washing and rinsing of the hair. Moreover, the shampoo exhibited excellent conditioning effect, and ensured excellent slipperiness and flexibility at the time of rinsing and after drying.

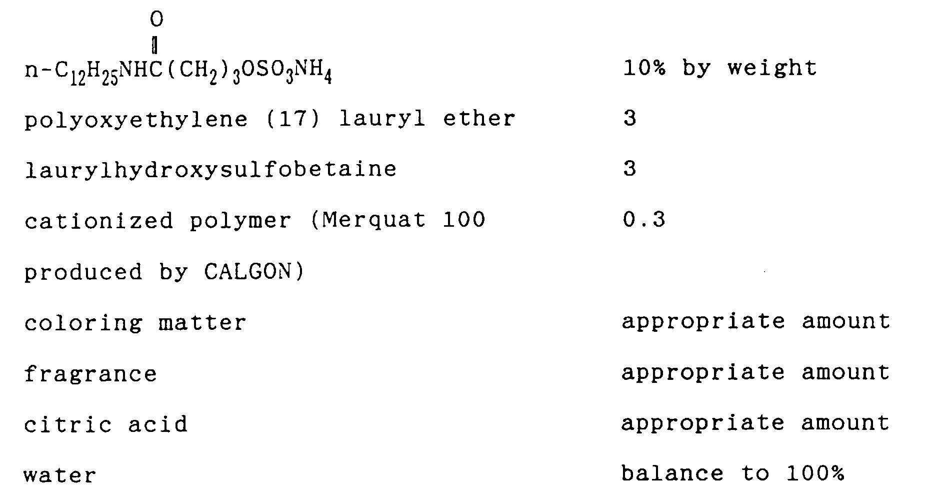

### Formulation Example 16

A shampoo having the composition given below was prepared. The obtained shampoo not only was excellent in foaming power and detergency, but also ensured good feeling without squeak at the time of washing and rinsing of the hair. Moreover, the shampoo exhibited excellent conditioning effect, and ensured excellent slipperiness and flexibility at the time of rinsing and after drying.

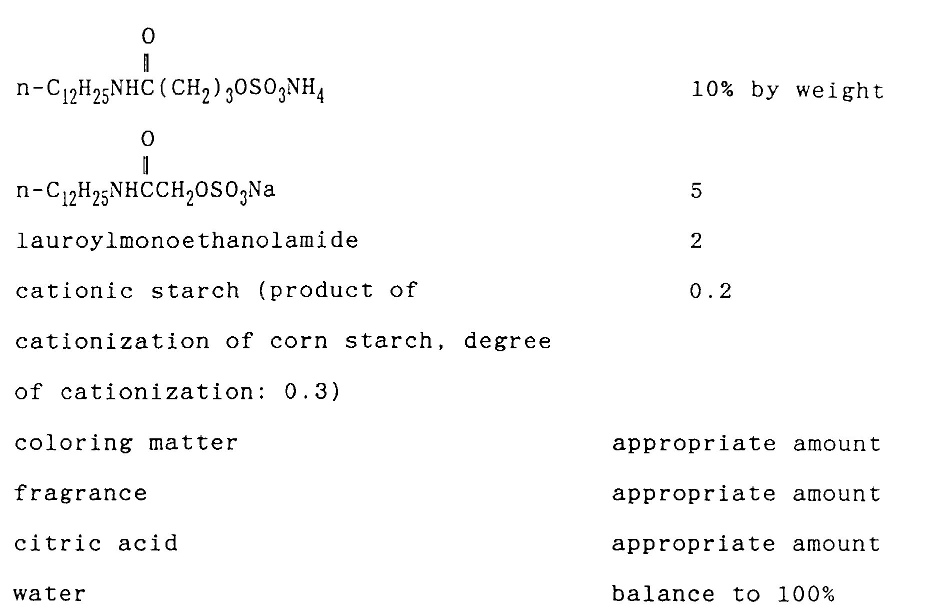

### Formulation Examples 17 to 24 and Comparative Formulation Examples 1 to 3

Various detergents for tableware each having the composition specified in Table 3 were prepared, and the foaming properties thereof were evaluated according to the following method. The results are also shown in Table 3.

### <Foaming test>

To deionized water at 25°C, each of the detergents for tableware together with butter was added to prepare an aqueous solution. The concentrations of the detergent and the butter in the solution were 5% by weight and 0.2% by weight, respectively. The aqueous solution was stirred by the reversing stirring technique at 1000 rpm for 5 min. The height of the formed foam remaining in 30 sec after the termination of the stirring was measured.

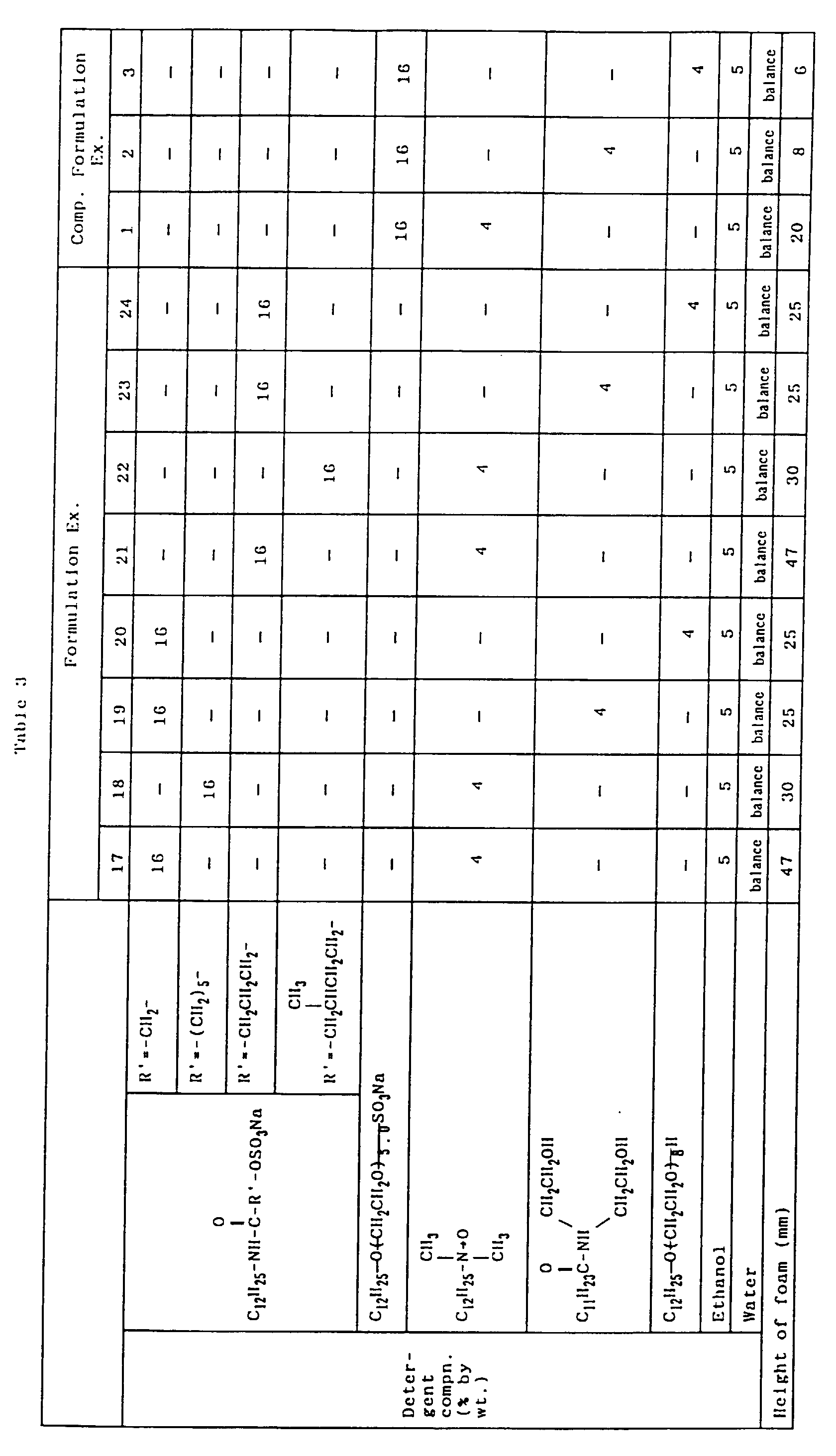

### Formulation Example 25 (A shampoo composition)

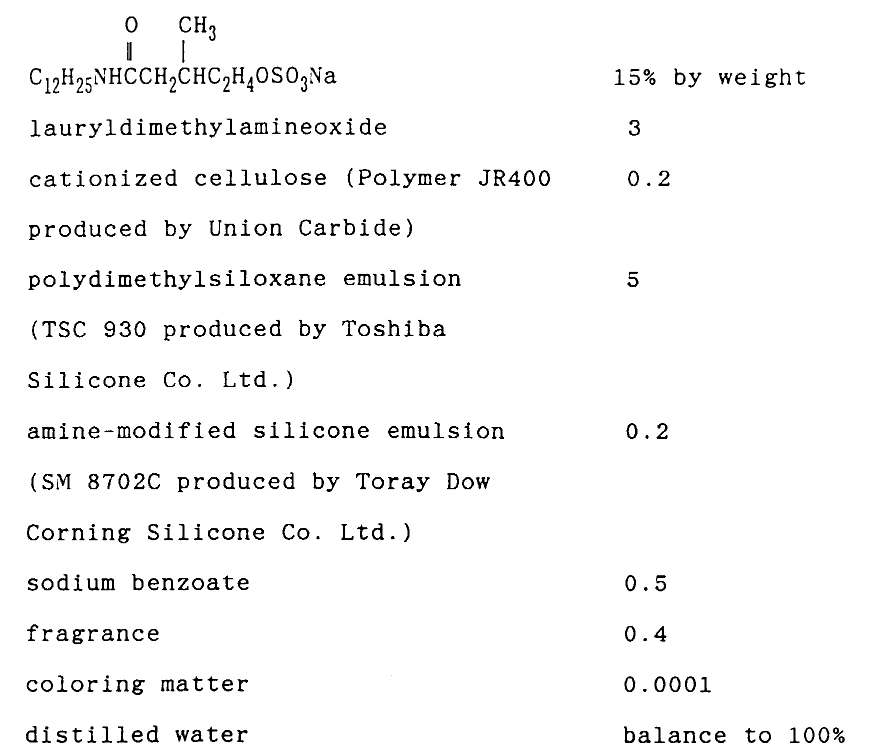

### Formulation Example 26 (A shampoo composition)

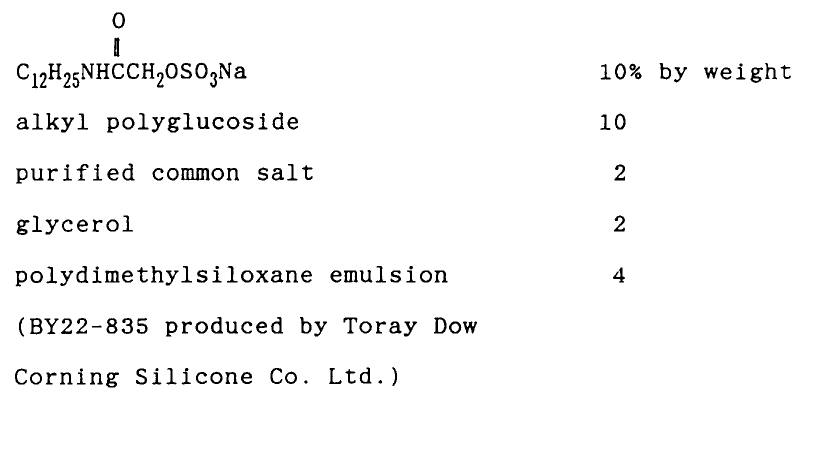

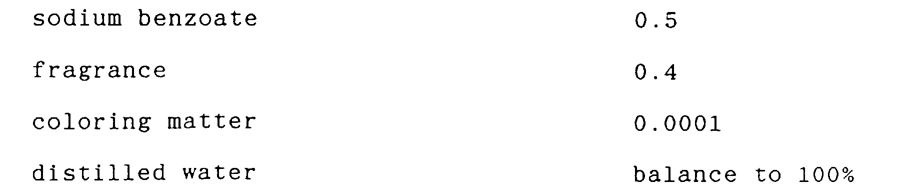

### Formulation Example 27 (A shampoo composition)

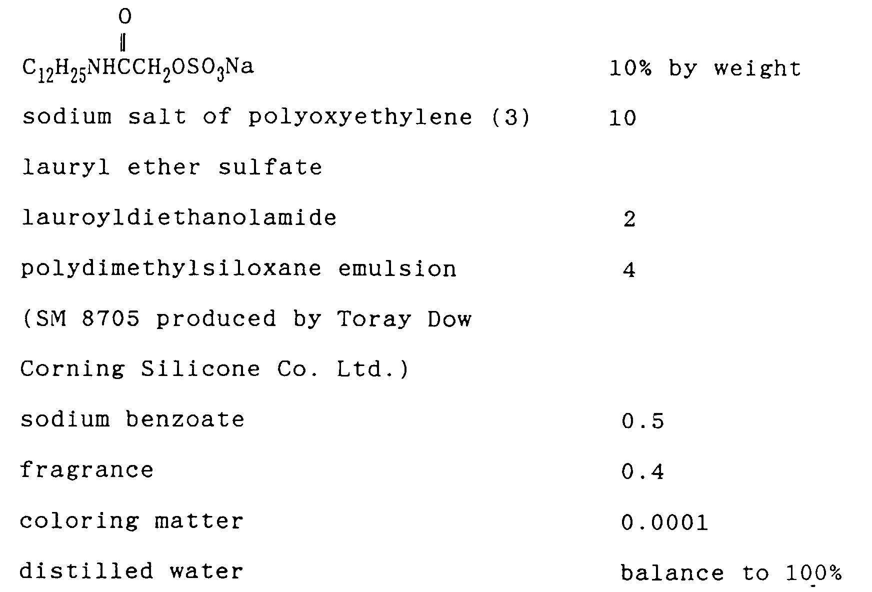

### Formulation Example 28 (A shampoo composition)

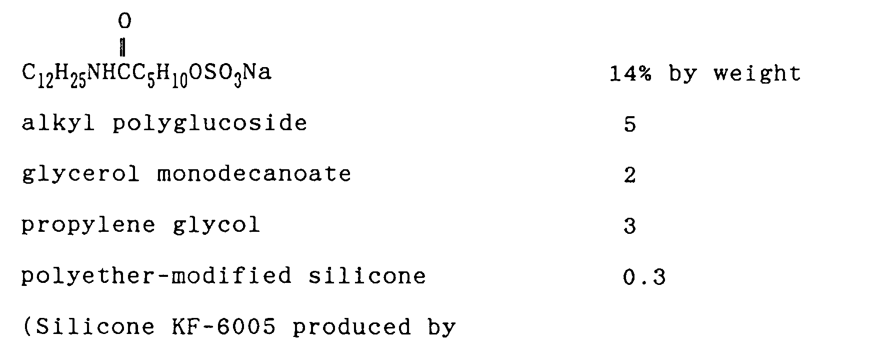

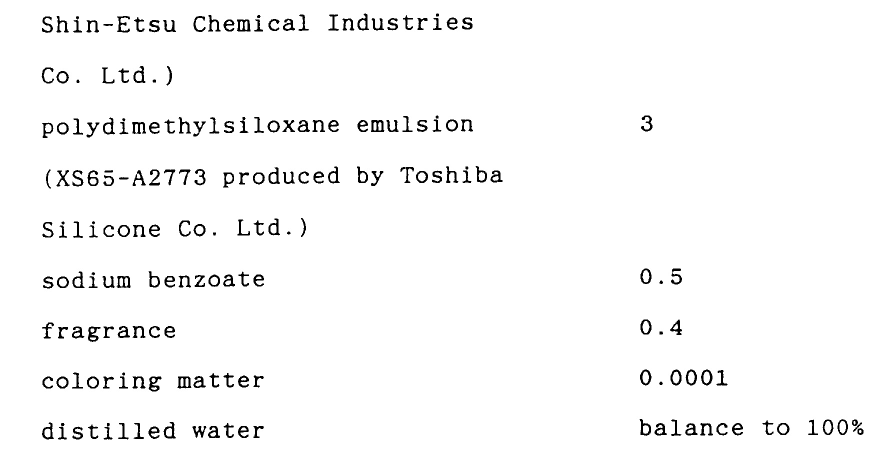

## Claims

1. An N-alkylcarbamylalkanol sulfate or a salt thereof represented by the general formulae (1-1) or (1-2): 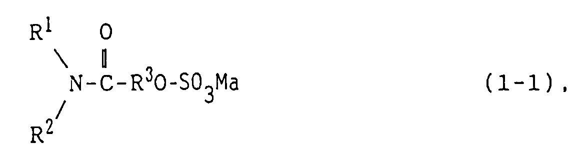 and 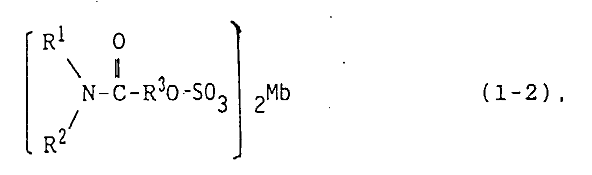 wherein R¹ represents a linear or branched alkyl or alkenyl group having 6 to 22 carbon atoms; R² represents a methyl group or a hydrogen atom; R³ represents a linear or branched alkylene group having 1 to 5 carbon atoms; Ma represents a hydrogen atom, an alkali metal atom, an ammonium group, an alkanolammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group, or a group consisting of a basic amino acid and a hydrogen atom; and Mb represents an alkaline earth metal atom; under the proviso that a compound of the formula (1-1) with R¹ being represented by a C₁₉ alkyl group of the formula 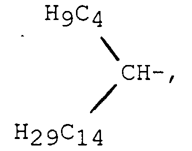 R² being represented by a hydrogen atom, R³ being represented by a methylene group and Ma being represented by a sodium atom is excluded.

2. The N-alkylcarbamylalkanol sulfate or the salt thereof according to claim 1, which is represented by the general formula (1-1) wherein R¹ represents a linear alkyl group having 8 to 18 carbon atoms; R² represents a hydrogen atom or a methyl group; R³ represents a linear alkylene group having 1 to 5 carbon atoms; and Ma represents an ammonium group, sodium or a triethanolammonium group.

3. The N-alkylcarbamylalkanol sulfate or the salt thereof according to claim 1, which is represented by the general formula (1-1) wherein R¹ represents a linear alkyl group having 8 to 18 carbon atoms; R² represents a hydrogen atom; R³ represents a linear or branched alkylene group having 1 to 5 carbon atoms; and Ma represents an ammonium group, sodium or a triethanolammonium group.

4. The N-alkylcarbamylalkanol sulfate or the salt thereof according to claim 1, which is represented by the general formula (1-1) wherein R¹ represents a linear alkyl group having 8 to 18 carbon atoms; R² represents a hydrogen atom; R³ represents a methylene group, a pentamethylene group or a 2-methylbutylene group; and Ma represents an ammonium group, sodium or a triethanolammonium group.

5. The N-alkylcarbamylalkanol sulfate or the salt thereof according to claim 4, which is represented by the general formula (1-1) wherein R³ represents a methylene group.

6. A process for producing the N-alkylcarbamylalkanol sulfate or the salt thereof of claim 1, which comprises conducting steps selected from A to C in an order selected from the group consisting of (1) A and B, and (2) C and B:
step A
Reacting an aliphatic amine represented by the general formula (2): 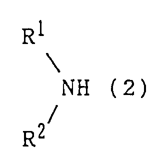 (wherein R¹ and R² are defined above), with a cyclic lactone represented by the general formula (3): 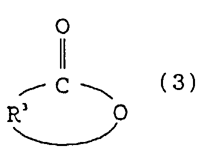 (wherein R³ is defined above), to thereby obtain an N-alkylcarbamylalkanol represented by the general formula (4): 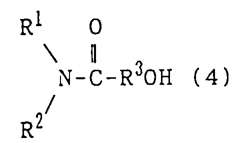 (wherein R¹, R² and R³ are defined above);
step B
Reacting the N-alkylcarbamylalkanol represented by the above general formulae (4) with a sulfating agent to thereby obtain an N-alkylcarbamylalkanol sulfate represented by the above general formula (1-1) (with the proviso that Ma is a hydrogen atom), and, if necessary, neutralizing the N-alkylcarbamylalkanol sulfate thus obtained with a basic compound to thereby obtain a salt of N-alkylcarbamylalkanol sulfate represented by the above general formulae (1-1) (with the proviso that Ma is not a hydrogen atom) or (1-2); and
step C
Reacting the aliphatic amine represented by the above general formula (2) with an ω-hydroxycarboxylic acid or an ester thereof represented by the general formula (6): HO-R³CO₂R⁵ (6) (wherein R³ is defined above, and R⁵ represents a hydrogen atom or a linear or branched alkyl group having 1 to 4 carbon atoms) to thereby obtain an N-alkylcarbamylalkanol represented by the above general formula (4).

7. The process for producing the N-alkylcarbamylalkanol sulfate or the salt thereof according to claim 6, wherein step C comprises reacting the aliphatic amine represented by the above general formula (2) with an ω-hydroxycarboxylic acid represented by the general formula (6'): HO-R³CO₂H (6') (wherein R³ is defined above) to thereby obtain an N-alkylcarbamylalkanol represented by the above general formula (4).

8. The process for producing the N-alkylcarbamylalkanol sulfate or the salt thereof according to claim 6, wherein the sulfating agent used in step B is selected from the group consisting of chlorosulfonic acid, sulfuric anhydride, fuming sulfuric acid, concentrated sulfuric acid and sulfamic acid.

9. Use of the N-alkylcarbamylalkanol sulfate or a salt thereof according to claims 1 to 5 as a component of a detergent composition.

10. A detergent composition comprising the N-alkyl-carbamylalkanol sulfate or a salt thereof according to claims 1 to 5.

11. The detergent composition according to claim 10, wherein the content of the N-alkylcarbamylalkanol sulfate or the salt thereof is 1 to 70% by weight based on the entire weight of the detergent composition.

12. The detergent composition according to claim 10, which further comprises at least one component selected from the group consisting of a silicone derivative, a cationic surfctant, a water-soluble cationic polymer, an anionic surfactant, a nonionic surfactant, an amphoteric surfactant, a humectant, a bactericide, an emulsifier, a fragrance, a coloring matter and water.

13. A detergent composition comprising the following components (A) and (B):
(A) 1 to 70% by weight, based on the total weight of the composition, of N-alkylcarbamylalkanol sulfate or a salt thereof represented by the general formula (2-1) or (2-2) 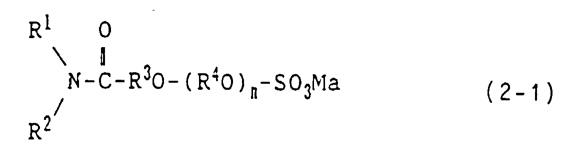 and 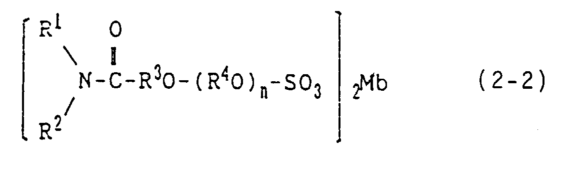 wherein R¹ represents a linear or branched alkyl or alkenyl group having 6 to 22 carbon atoms; R² represents a C₁-C₂₂ alkyl group, a C₂-C₂₂ alkenyl group or a hydrogen atom; R³ represents a linear or branched alkylene group having 1 to 5 carbon atoms; R⁴O represents an oxyalkylene group having 2 or 3 carbon atoms; n represents an average addition mole number of the oxyalkylene group and is a number between 0 and 20, and wherein each R⁴O group may be the same or different from one another; Ma represents a hydrogen atom, an alkali metal atom, an ammonium group, an alkanol-ammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group, or a group consisting of a basic amino acid and a hydrogen atom; and Mb represents an alkaline earth metal atom;
(B) 0.01 to 3% by weight, based on the total weight of the composition, of a silicone derivative.

14. A detergent composition comprising the following components (A') and (B'):
(A') 1 to 70% by weight, based on the total weight of the composition, of N-alkylcarbamylalkanol sulfate or a salt thereof represented by the general formula (2-1) or (2-2) 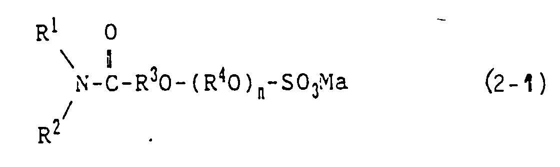 and 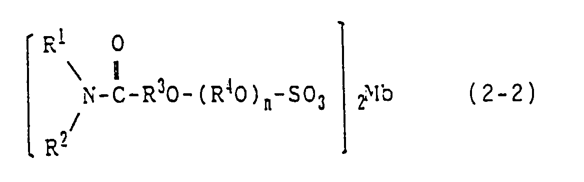 wherein R¹ represents a linear or branched alkyl or alkenyl group having 6 to 22 carbon atoms; R² represents a C₁-C₂₂ alkyl group, a C₂-C₂₂ alkenyl group or a hydrogen atom; R³ represents a linear or branched alkylene group having 1 to 5 carbon atoms; R⁴O represents an oxyalkylene group having 2 or 3 carbon atoms; n represents an average addition mole number of the oxyalkylene group and is a number between 0 and 20, and wherein each R⁴O group may be the same or different from one another; Ma represents a hydrogen atom, an alkali metal atom, an ammonium group, an alkanol-ammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group, or a group consisting of a basic amino acid and a hydrogen atom; and Mb represents an alkaline earth metal atom;
(B') 0.05 to 10% by weight, based on the total weight of the composition, of a cationic surfactant.

15. A detergent composition comprising the following components (A") and (B").
(A") 1 to 70% by weight, based on the total weight of the composition, of N-alkylcarbamylalkanol sulfate or a salt thereof represented by the general formula (2-1) or (2-2) 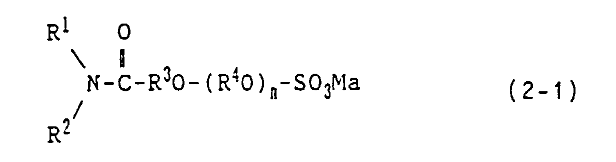 and 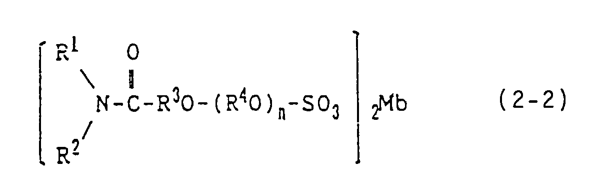 wherein R¹ represents a linear or branched alkyl or alkenyl group having 6 to 22 carbon atoms; R² represents a C₁-C₂₂ alkyl group, a C₂-C₂₂ alkenyl group or a hydrogen atom; R³ represents a linear or branched alkylene group having 1 to 5 carbon atoms; R⁴O represents an oxyalkylene group having 2 or 3 carbon atoms; n represents an average addition mole number of the oxyalkylene group and is a number between 0 and 20, and wherein each R⁴O group may be the same or different from one another; Ma represents a hydrogen atom, an alkali metal atom, an ammonium group, an alkanol-ammonium group having 2 to 9 carbon atoms in total, an alkylammonium group having 1 to 22 carbon atoms in total, an alkenylammonium group having 2 to 22 carbon atoms in total, a C₁-C₁₈ alkyl- or C₂-C₁₈ alkenyl-substituted pyridinium group, or a group consisting of a basic amino acid and a hydrogen atom; and Mb represents an alkaline earth metal atom;
(B") 0.01 to 3% by weight, based on the total weight of the composition, of a water-soluble cationic polymer.

## Patentansprüche

1. N-Alkylcarbamylalkanolsulfat oder ein Salz davon, dargestellt durch die allgemeinen Formeln (1-1) oder (1-2): 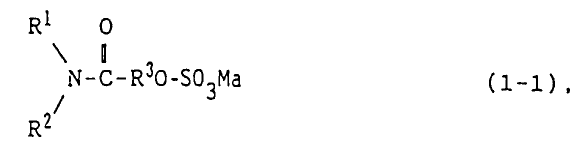 und 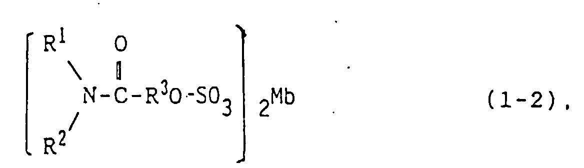 worin R¹ eine lineare oder verzweigte Alkyl- oder Alkenylgruppe mit 6 bis 22 Kohlenstoffatomen ist; R² eine Methylgruppe oder ein Wasserstoffatom ist; R³ eine lineare oder verzweigte Alkylengruppe mit 1 bis 5 Kohlenstoffatomen ist, Ma ein Wasserstoffatom, ein Alkalimetallatom, eine Ammoniumgruppe, eine Alkanolammoniumgruppe mit insgesamt 2 bis 9 Kohlenstoffatomen, eine Alkylammoniumgruppe mit insgesamt 1 bis 22 Kohlenstoffatomen, eine Alkenylammoniumgruppe mit insgesamt 2 bis 22 Kohlenstoffatomen, eine C₁₋₁₈-Alkyl- oder C₂₋₁₈-Alkenyl- substituierte Pyridiniumgruppe oder eine Gruppe, bestehend aus einer basischen Aminosäure und einem Wasserstoffatom, ist; und Mb ein Erdalkalimetallatom ist; mit dem Vorbehalt, daß eine Verbindung der Formel (1-1) ausgeschlossen ist, worin R¹ eine C₁₉-Alkylgruppe der Formel ist, 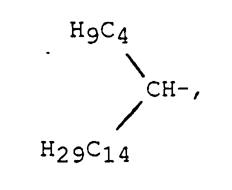 R² ein Wasserstoffatom ist, R³ eine Methylengruppe und Ma ein Natriumatom ist.

2. N-Akylcarbamylalkanolsulfat oder das Salz davon nach Anspruch 1, das durch die allgemeine Formel (1-1) dargestellt ist, worin R¹ eine lineare Alkylgruppe mit 8 bis 18 Kohlenstoffatomen ist; R² ein Wasserstoffatom oder eine Methylgruppe ist; R³ eine lineare Alkylengruppe mit 1 bis 5 Kohlenstoffatomen ist; und Ma eine Ammoniumgruppe, Natrium oder Triethanolammoniumgruppe ist.

3. N-Alkylcarbamylalkanolsulfat oder das Salz davon nach Anspruch 1, das durch die allgemeine Formel (1-1) dargestellt ist, worin R¹ eine lineare Alkylgruppe mit 8 bis 18 Kohlenstoffatomen ist; R² ein Wasserstoffatom ist; R³ ein lineare oder verzweigte Alkylengruppe mit 1 bis 5 Kohlenstoffatomen ist; und Ma eine Ammoniumgruppe, Natrium oder eine Triethanolammoniumgruppe ist.

4. N-Alkylcarbamylalkanolsulfat oder das Salz davon nach Anspruch 1, das durch die allgemeine Formel (1-1) dargestellt ist, worin R¹ eine lineare Alkylgruppe mit 8 bis 18 Kohlenstoffatomen ist; R² ein Wasserstoffatom ist; R³ eine Methylengruppe, eine Pentamethylengruppe oder eine 2-Methylbutylengruppe ist; und Ma eine Ammoniumgruppe, Natrium oder eine Triethanolammoniumgruppe ist.

5. N-Alkylcarbamylalkanolsulfat oder das Salz davon nach Anspruch 4, das durch die allgemeine Formel (1-1) dargestellt ist, worin R³ eine Methylengruppe ist.

6. Verfahren zur Erzeugung des N-Alkylcarbamylalkanolsulfates oder des Salzes davon nach Anspruch 1, umfassend die Durchführung von Schritten, ausgewählt aus A bis C in einer Reihenfolge, ausgewählt aus der Gruppe bestehend aus (1) A und B, und (2) C und B:
Schritt A
Reaktion eines aliphatischen Amins, dargestellt durch die allgemeine Formel (2): 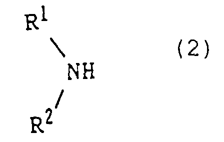 (worin R¹ und R² wie oben definiert sind), mit einem zyklischen Lacton, dargestellt durch die allgemeine Formel (3): 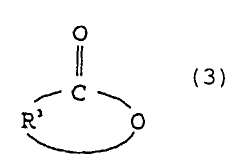 worin R³ wie oben definiert ist), unter Erhalt eines N-Alkylcarbamylalkanols, dargestellt durch die allgemeine Formel (4): 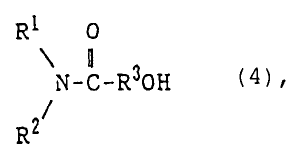 (worin R¹, R² und R³ wie oben definiert sind)
Schritt B:
Reaktion des N-Alkylcarbamylalkanols, dargestellt durch die obige allgemeine Formel (4) mit einem Sulfatierungsmittel, unter Erhalt eines N-Alkylcarbamylalkanolsulfates, dargestellt durch die obige allgemeine Formel (1-1) (mit dem Vorbehalt, daß Ma ein Wasserstoffatom ist) und gegebenenfalls Neutralisierung des somit erhaltenen N-Alkylcarbamylalkanolsulfates mit einer basischen Verbindung, unter Erhalt eines Salzes cdx N-Alkylcarbamylalkanolsulfates, dargestellt durch die obigen allgemeinen Formeln (1-1) (mit dem Vorbehalt, daß Ma kein Wasserstoffatom ist) oder (1-2); und
Schritt C:
Reaktion des aliphatischen Amins, dargestellt durch die obige allgemeine Formel (2) mit einer ω-Hydroxycarbonsäure oder einem Ester davon, dargestellt durch die allgemeine Formel (6): HO-R³CO₂R⁵ (6) (worin R³ wie oben definiert ist und R⁵ ein Wasserstoffatom oder eine lineare oder verzweigte Alkylgruppe mit 1 bis 4 Kohlenstoffatomen ist), unter Erhalt eines N-Alkylcarbamylalkanols, dargestellt durch die obige allgemeine Formel (4).

7. Verfahren zur Erzeugung des N-Alkylcarbamylalkanolsulfates oder des Salzes davon nach Anspruch 6, worin der Schritt C die Reaktion des aliphatischen Amins, dargestellt durch die obige allgemeine Formel (2), mit einer ω-Hydroxycarbonsäure, dargestellt durch die allgemeine Formel (6') HO-R³CO₂H (6') (worin R³ wie oben definiert ist) umfaßt, unter Erhalt eines N-Alkylcarbamylalkanols, dargestellt durch die obige allgemeine Formel (4).

8. Verfahren zur Erzeugung des N-Alkylcarbamylalkanolsulfates oder des Salzes davon nach Anspruch 6, worin das in dem Schritt B verwendete Sulfatierungsmittel ausgewählt ist aus der Gruppe, bestehend aus Chlorosulfonsäure, Schwefelsäureanhydrid, rauchende Schwefelsäure, konzentrierter Schwefelsäure und Sulfaminsäure.

9. Verwendung des N-Alkylcarbamylalkanolsulfates oder eines Salzes davon nach den Ansprüchen 1 bis 5 als eine Komponente für eine Detergenszusammensetzung.

10. Detergenszusammensetzung, umfassend das N-Alkylcarbamylalkanolsulfat oder ein Salz davon nach den Ansprüchen 1 bis 5.

11. Detergenszusammensetzung nach Anspruch 10, worin der Gehalt des N-Alkylcarbamylalkanolsulfates oder des Salzes davon 1 bis 70 Gew.% ist, bezogen auf das Gesamtgewicht der Detergenszusammensetzung.

12. Detergenszusammensetzung nach Anspruch 10, die weiterhin zumindest eine Komponente enthält, ausgewählt aus der Gruppe, bestehend aus einem Silikonderivat, einem kationischen Tensid, einem wasserlöslichen kationischen Polymer, einem anionischen Tensid, einem nicht-ionischen Tensid, einem amphoteren Tensid, einem Feuchtigkeitsmittel, einem Bakterizid, einem Emulgator, einem Duftstoff, einem Färbestoff und Wasser.

13. Detergenszusammensetzung, umfassend die folgenden Komponenten (A) und (B):
(A) 1 bis 70 Gew.% des N-Alkylcarbamylalkanolsulfats oder eines Salzes davon, dargestellt durch die allgemeinen Formeln (2-1) oder (2-2), bezogen auf das Gesamtgewicht der Zusammensetzung 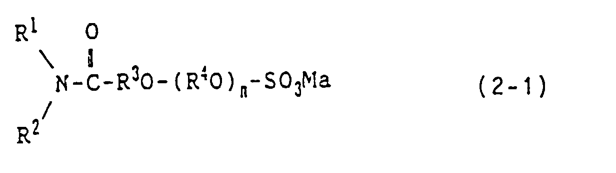 und 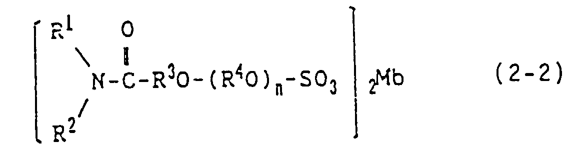 worin R¹ eine lineare oder verzweigte Alkyl- oder Alkenylgruppe mit 6 bis 22 Kohlenstoffatomen ist; R² eine C₁₋₂₂-Alkylgruppe, eine C₂₋₂₂-Alkenylgruppe oder ein Wasserstoffatom ist; R³ eine lineare oder verzweigte Alkylengruppe mit 1 bis 5 Kohlenstoffatomen ist; R⁴O eine Oxyalkylengruppe mit 2 oder 3 Kohlenstoffatomen ist; n eine durchschnittliche Additionsmolzahl der Oxyalkylengruppe bedeutet und eine Zahl zwischen 0 und 20 ist, und worin jede R⁴O Gruppe gleich oder verschieden voneinander sein kann; Ma ein Wasserstoffatom, ein Alkalimetallatom, eine Ammoniumgruppe, eine Alkanolammoniumgruppe mit insgesamt 2 bis 9 Kohlenstoffatomen, eine Alkylammoniumgruppe mit insgesamt 1 bis 22 Kohlenstoffatomen, eine Alkenylammoniumgruppe mit insgesamt 2 bis 22 Kohlenstoffatomen, eine C₁₋₁₈-Alkyl- oder c₂₋₁₈-Alkenyl- substituierte Pyridiniumgruppe oder eine Gruppe, bestehend aus einer basischen Aminosäure und einem Wasserstoffatom, bedeutet; und Mb ein Erdalkalimetallatom ist; (b) 0,01 bis 3 Gew.%, bezogen auf das Gesamtgewicht der Zusammensetzung, eines Silikonderivates.

14. Detergenszusammensetzung, umfassend die folgenden Komponenten (A') und (B'):
(A') 1 bis 70 Gew.%, bezogen auf das Gesamtgewicht der Zusammensetzung, des N-Alkylcarbamylalkanolsulfats oder eines Salzes davon, dargestellt durch die allgemeine Formel (2-1) oder (2-2) 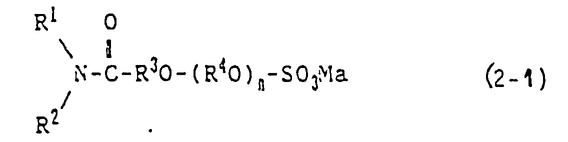 und 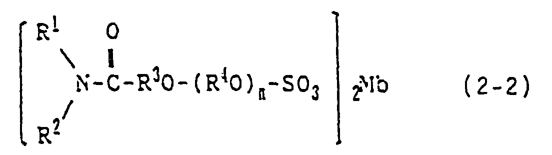 worin R¹ eine lineare oder verzweigte Alkyl- oder Alkenylgruppe mit 6 bis 22 Kohlenstoffatomen ist; R² eine C₁₋₂₂-Alkylgruppe, eine C₂₋₂₂-Alkenylgruppe oder ein Wasserstoffatom ist; R³ eine lineare oder verzweigte Alkylengruppe mit 1 bis 5 Kohlenstoffatomen ist; R⁴O eine Oxyalkylengruppe mit 2 oder 3 Kohlenstoffatomen ist; n eine durchschnittliche Additionsmolzahl der Oxyalkylengruppe ist und eine Zahl zwischen 0 und 20 ist, und worin jede R⁴O-Gruppe gleich oder verschieden voneinander sein kann; Ma ein Wasserstoffatom, ein Alkalimetallatom, eine Ammoniumgruppe, eine Alkanolammoniumgruppe mit insgesamt 2 bis 9 Kohlenstoffatomen, eine Alkylammoniumgruppe mit insgesamt 1 bis 22 Kohlenstoffatomen, eine Alkenylammoniumgruppe mit insgesamt 2 bis 22 Kohlenstoffatomen, eine C₁₋₂₈-Alkyl- oder C₂₋₁₈-Alkenyl-substituierte Pyridiniumgruppe ist, oder eine Gruppe ist, bestehend aus einer basischen Aminosäure oder einem Wasserstoffatom; und Mb ein Erdalkalimetallatom ist;
(B') 0,05 bis 10 Gew.%, bezogen auf das Gesamtgewicht der Zusammensetzung, eines kationischen Tensides.

15. Detergenszusammensetzung, umfassend die folgenden Komponenten (A") und (B"):
(A") 1 bis 70 Gew.%, bezogen auf das Gesamtgewicht der Zusammensetzung, des N-Alkylcarbamylalkanolsulfats oder eines Salzes davon, dargestellt durch die allgemeinen Formeln (2-1) oder (2-2) 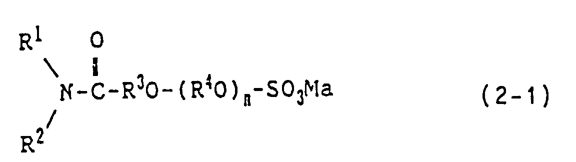 und 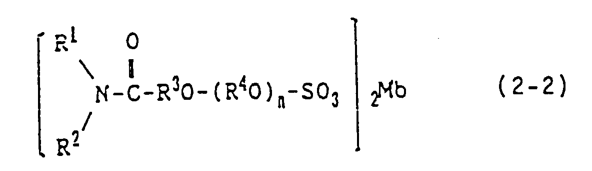 worin R¹ eine lineare oder verzweigte Alkyl- oder Alkenylgruppe mit 6 bis 22 Kohlenstoffatomen ist; R² ein C₁₋₂₂-Alkylgruppe, eine C₂₋₂₂-Alkenylgruppe oder ein Wasserstoffatom ist; R³ eine lineare oder verzweigte Alkylengruppe mit 1 bis 5 Kohlenstoffatomen ist; R⁴O eine Oxyalkylengruppe mit 2 oder 3 Kohlenstoffatomen ist; n eine durchschnittliche Additionsmolzahl der Oxyalkylengruppe bedeutet und eine Zahl zwischen 0 und 20 ist, und worin jede R⁴O-Gruppe gleich oder verschieden voneinander sein kann; Ma ein Wasserstoffatom, ein Alkalimetallatom, eine Ammoniumgruppe, ein Alkanolammoniumgruppe mit insgesamt 2 bis 9 Kohlenstoffatomen, eine Alkylammoniumgruppe mit insgesamt 1 bis 22 Kohlenstoffatomen, eine Alkenylammoniumgruppe mit insgesamt 2 bis 22 Kohlenstoffatomen, eine C₁₋₁₈-Alkyl- oder C₂₋₁₈-Alkenyl-substituierte Pyridiniumgruppe oder eine Gruppe ist, bestehend aus einer basischen Aminosäure und einem Wasserstoffatom; und Mb ein Erdalkaliatom ist;
(B") 0,01 bis 3 Gew.%, bezogen auf das Gesamtgewicht der Zusammensetzung, eines wasserlöslichen kationischen Polymers.

## Revendications

1. Sulfate de N-alkylcarbamylalcanol, ou l'un de ses sels, représenté par les formules générales (1-1) ou (1-2) : 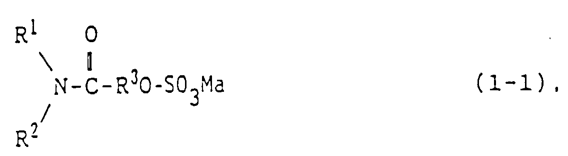 et 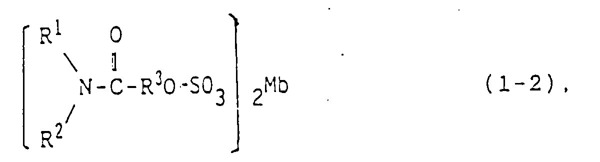 où R¹ représente un groupe alkyle ou alcényle linéaire ou ramifié comportant 6 à 22 atomes de carbone ; R² représente un groupe méthyle ou un atome d'hydrogène ; R³ représente un groupe alkylène linéaire ou ramifié comportant 1 à 5 atomes de carbone ; Ma représente un atome d'hydrogène, un atome d'un métal alcalin, un groupe ammonium, un groupe alcanolammonium comportant un total de 2 à 9 atomes de carbone, un groupe alkylammonium comportant un total de 1 à 22 atomes de carbone, un groupe alcénylammonium comportant un total de 2 à 22 atomes de carbone, un groupe pyridinium substitué par un groupe alkyle en C₁ à C₁₈ ou par un groupe alcényle en C₂ à C₁₈, ou un groupe constitué d'un acide aminé basique et d'un atome d'hydrogène et Mb représente un atome d'un métal alcalino-terreux ; à la condition qu'un composé de la formule (1-1), avec R¹ étant représenté par un groupe alkyle en C₁₉ de la formule 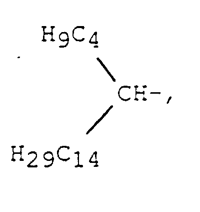 R² étant représenté par un atome d'hydrogène, R³ étant représenté par un groupe méthylène et Ma étant représenté par un atome de sodium, soit exclu.

2. Sulfate de N-alkylcarbamylalcanol, ou son sel, selon la revendication 1, qui est représenté par la formule générale (1-1) où R¹ représente un groupe alkyle linéaire comportant 8 à 18 atomes de carbone ; R² représente un atome d'hydrogène ou un groupe méthyle ; R³ représente un groupe alkylène linéaire comportant 1 à 5 atomes de carbone et Ma représente un groupe ammonium, un atome de sodium ou un groupe triéthanolammonium.

3. Sulfate de N-alkylcarbamylalcanol, ou son sel, selon la revendication 1, qui est représenté par la formule générale (1-1) où R¹ représente un groupe alkyle linéaire comportant 8 à 18 atomes de carbone ; R² représente un atome d'hydrogène ; R³ représente un groupe alkylène linéaire ou ramifié comportant 1 à 5 atomes de carbone et Ma représente un groupe ammonium, un atome de sodium ou un groupe triéthanolammonium

4. Sulfate de N-alkylcarbamylalcanol, ou son sel, selon la revendication 1, qui est représenté par la formule générale (1-1) où R¹ représente un groupe alkyle linéaire comportant 8 à 18 atomes de carbone ; R² représente un atome d'hydrogène ; R³ représente un groupe méthylène, un groupe pentaméthylène ou un groupe 2-méthylbutylène et Ma représente un groupe ammonium, un atome de sodium ou un groupe triéthanolammonium.

5. Sulfate de N-alkylcarbamylalcanol, ou son sel, selon la revendication 4, qui est représenté par la formule générale (1-1) où R³ représente un groupe méthylène.

6. Procédé de production du sulfate de N-alkylcarbamylalcanol, ou de son sel, selon la revendication 1, qui comprend la mise en oeuvre des étapes sélectionnées de A à C selon un ordre sélectionné dans le groupe constitué de (1) A et B et (2) C et B :
Etape A
On fait réagir une amine aliphatique représentée par la formule générale (2) : 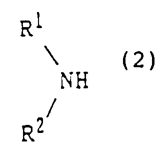 (où R¹ et R² sont définis ci-dessus) avec une lactone cyclique représentée par la formule générale (3) : 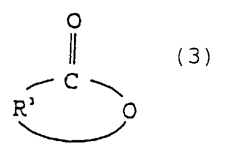 (où R³ est défini ci-dessus) pour obtenir, ainsi, un N-alkylcarbamylalcanol représenté par la formule générale (4) : 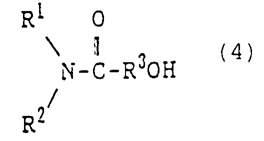 (où R¹, R² et R³ sont définis ci-dessus);
Etape B
On fait réagir le N-alkylcarbamylalcanol représenté par les formules générales (4) ci-dessus avec un agent de sulfatation pour obtenir, ainsi, un sulfate de N-alkylcarbamylalcanol représenté par la formule générale (1-1) ci-dessus (à la condition que Ma soit un atome d'hydrogène) et, si nécessaire, on neutralise le sulfate de N-alkylcarbamylalcanol ainsi obtenu avec un composé basique pour obtenir ainsi un sel de sulfate de N-alkylcarbamylalcanol représenté par les formules générales (1-1) (à la condition que Ma ne soit pas un atome d'hydrogène) ou (1-2) ci-dessus, et
Etape C
On fait réagir l'amine aliphatique représentée par la formule générale (2) ci-dessus avec un acide ω-hydroxycarboxylique, ou l'un de ses esters, représenté par la formule générale (6) : OH-R³CO₂R⁵ (6) (où R³ est défini ci-dessus et R⁵ représente un atome d'hydrogène ou un groupe alkyle linéaire ou ramifié comportant 1 à 4 atomes de carbone) pour obtenir ainsi un N-alkylcarbamylalcanol représenté par la formule générale (4) ci-dessus.

7. Procédé de production du sulfate de N-alkylcarbamylalcanol, ou de son sel, selon la revendication 6, dans lequel l'étape C comrend le fait de faire réagir l'amine aliphatique représentée par la formule générale (2) ci-dessus avec un acide ω-hydroxycarboxylique représenté par la formule générale (6') : HO-R³CO₂H (6') (où R³ est défini ci-dessus) pour obtenir, ainsi, un N-alkylcarbamylalcanol représenté par la formule générale (4) ci-dessus.

8. Procédé de production du sulfate de N-alkylcarbamylalcanol, ou de son sel, selon la revendication 6, dans lequel l'agent de sulfatation que l'on utilise à l'étape B est sélectionné dans le groupe constitué d'acide chlorosulfonique, d'anhydride sulfurique, d'acide sulfurique fumant, d'acide sulfurique concentré et d'acide sulfamique.

9. Utilisation du sulfate de N-alkylcarbamylalcanol, ou de l'un de ses sels, selon les revendications 1 à 5 comme constituant d'une composition détergente.

10. Composition détergente comprenant le sulfate de N-alkylcarbamylalcanol, ou l'un de ses sels, selon les revendications 1 à 5.

11. Composition détergente selon la revendication 10, dans laquelle la teneur en sulfate du N-alkylcarbamylalcanol, ou en l'un de ses sels, est de 1 à 70 % en poids sur la base du poids entier de la composition détergente.

12. Composition détergente selon la revendication 10 qui comprend, en outre, au moins un constituant sélectionné dans le groupe constitué d'un dérivé de la silicone, d'un agent tensio-actif cationique, d'un polymère cationique hydrosoluble, d'un agent tensio-actif anionique, d'un agent tensio-actif non ionique, d'un agent tensio-actif amphotère, d'un agent mouillant, d'un bactéricide, d'un émulsifiant, d'une fragrance, d'une matière colorante et d'eau.

13. Composition détergente comprenant les constituants (A) et (B) suivants :
(A) 1 à 70 % en poids, sur la base du poids total de la composition, de sulfate de N-alkylcarbamylalcanol ou de l'un de ses sels représenté par la formule générale (2-1) ou (2-2) 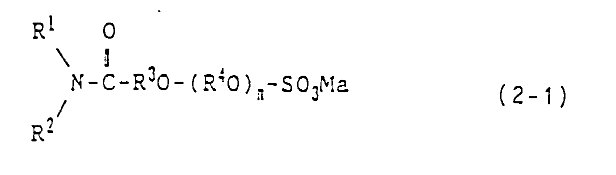 et 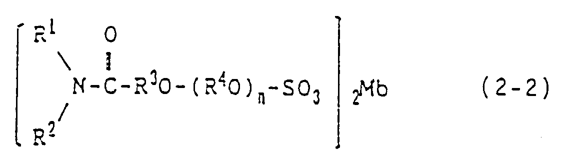 où R¹ représente un groupe alkyle ou alcényle linéaire ou ramifié comportant 6 à 22 atomes de carbone ; R² représente un groupe alkyle en C₁ à C₂₂, un groupe alcényle en C₂ à C₂₂ ou un atome d'hydrogène ; R³ représente un groupe alkylène linéaire ou ramifié comportant 1 à 5 atomes de carbone ; R⁴O représente un groupe oxyalkylène comportant 2 ou 3 atomes de carbone ; n représente un nombre moyen de moles d'addition du groupe oxyalkylène et est un nombre situé entre 0 et 20 et où chacun des groupes R⁴O peut être identique ou différent d'un groupe à l'autre ; Ma représente un atome d'hydrogène, un atome d'un métal alcalin, un groupe ammonium, un groupe alcanolammonium comportant un total de 2 à 9 atomes de carbone, un groupe alkylammonium comportant un total de 1 à 22 atomes de carbone, un groupe alcénylammonium comportant un total de 2 à 22 atomes de carbone, un groupe pyridinium substitué par un groupe alkyle en C₁ à C₁₈ ou par un groupe alcényle en C₂ à C₁₈, ou un groupe constitué d'un acide aminé basique et d'un atome d'hydrogène, et Mb représente un atome d'un métal alcalino-terreux.
(B) 0,01 à 3 % en poids, sur la base du poids total de la composition, d'un dérivé de la silicone.

14. Composition détergente comprenant les constituants (A') et (B') suivants :
(A') 1 à 70 % en poids, sur la base du poids total de la composition, de sulfate de N-alkylcarbamylalcanol, ou de l'un de ses sels, représenté par la formule générale (2-1) ou (2-2) 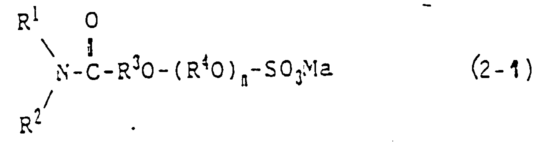 et 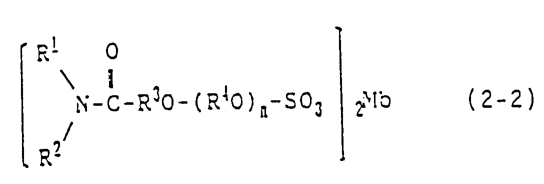 où R¹ représente un groupe alkyle ou alcényle linéaire ou ramifié comportant 6 à 22 atomes de carbone ; R² représente un groupe alkyle en C₁ à C₂₂, un groupe alcényle en C₂ à C₂₂ ou un atome d'hydrogène ; R³ représente un groupe alkylène linéaire ou ramifié comportant 1 à 5 atomes de carbone ; R⁴O représente un groupe oxyalkylène comportant 2 ou 3 atomes de carbone ; n représente un nombre moyen de moles d'addition du groupe oxyalkylène et est un nombre compris entre 0 et 20, et où chaque groupe R⁴O peut être identique ou différent d'un groupe à l'autre ; Ma représente un atome d'hydrogène, un atome d'un métal alcalin, un groupe ammonium, un groupe alcanolammonium comportant un total de 2 à 4 atomes de carbone, un groupe alkylammonium comportant un total de 1 à 22 atomes de carbone, un groupe alcénylammonium comportant un total de 2 à 22 atomes de carbone, un groupe pyridinium substitué par un groupe alkyle en C₁ à C₁₈, ou par un groupe alcényle en C₂ à C₁₈ ou un groupe constitué d'un acide aminé basique et d'un atome d'hydrogène, et Mb représente un atome d'un métal alcalino-terreux ;
(B') 0,05 à 10 % en poids, sur la base du poids total de la composition, d'un agent tensio-actif cationique.

15. Composition détergente comprenant les constituants (A") et (B") suivants :
(A") 1 à 70 % en poids, sur la base du poids total de la composition, de sulfate de N-alkylcarbamylalcanol, ou de l'un de ses sels, représenté par la formule générale (2-1) ou (2-2) 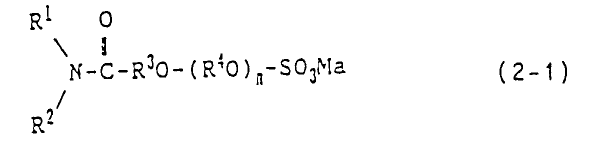 et 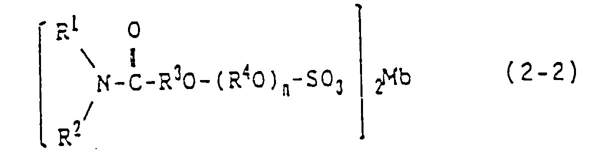 où R¹ représente un groupe alkyle ou alcényle linéaire ou ramifié comportant 6 à 22 atomes de carbone ; R² représente un groupe alkyle en C₁ à C₂₂, un groupe alcényle en C₂ à C₂₂ ou un atome d'hydrogène ; R³ représente un groupe alkylène linéaire ou ramifié comportant 1 à 5 atomes de carbone ; R⁴O représente un groupe oxyalkylène comportant 2 ou 3 atomes de carbone ; n représente un nombre moyen de moles d'addition du groupe oxyalkylène et est un nombre compris entre 0 et 20, et où chaque groupe R⁴O peut être identique ou différent d'un groupe à l'autre ; Ma représente un atome d'hydrogène, un atome d'un métal alcalin, un groupe ammonium, un groupe alcanolammonium comportant un total de 2 à 9 atomes de carbone, un groupe alkylammonium comportant un total de 1 à 22 atomes de carbone, un groupe alcénylammonium comportant un total de 2 à 22 atomes de carbone, un groupe pyridinium substitué par un groupe alkyle en C₁ à C₁₈, ou par un groupe alcényle en C₂ à C₁₈ ou un groupe constitué d'un acide aminé basique et d'un atome d'hydrogène, et Mb représente un atome d'un métal alcalino-terreux ;
(B") 0,01 à 3 % en poids, sur la base du poids total de la composition, d'un polymère cationique hydrosoluble.
